(19) Europäisches Patentamt / European Patent Office / Office européen des brevets

(11) **EP 4 555 939 A2**

(12) **EUROPEAN PATENT APPLICATION**

(43) Date of publication:
**21.05.2025** Bulletin 2025/21

(21) Application number: **25168408.0**

(22) Date of filing: **27.01.2021**

(51) International Patent Classification (IPC):
***A61B 8/00*** (2006.01)

(52) Cooperative Patent Classification (CPC):
**A61B 5/022; A61B 8/04; A61B 8/0858;
A61B 8/4494; A61B 8/5223;** A61B 5/02133;
A61B 5/14551; A61B 8/0891

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.01.2020 US 202062966927 P**

(62) Document number(s) of the earlier application(s) in
accordance with Art. 76 EPC:
**21747357.8 / 4 096 510**

(71) Applicant: **California Institute of Technology
Pasadena, CA 91125 (US)**

(72) Inventors:
• **RAJAGOPAL, Aditya
Pasadena, 91125 (US)**
• YURK, Dominic
**Pasadena, 91125 (US)**
• ABU-MOSTAFA, Yaser
**Pasadena, 91125 (US)**
• RAJAGOPAL, Alaina Ann Brinley
**Pasadena, 91125 (US)**
• JIMENEZ, Raymond
**Orange, 92865 (US)**

(74) Representative: **Marks & Clerk LLP
15 Fetter Lane
London EC4A 1BW (GB)**

Remarks:
This application was filed on 03-04-2025 as a
divisional application to the application mentioned
under INID code 62.

(54) **BLOOD PRESSURE MEASUREMENT APPARATUS AND METHODS OF USE THEREOF**

(57) Provided are an apparatus and method for blood pressure measurement using an electroacoustic transducer in combination with a piezoelectric ultrasonic transducer. The apparatus and method can provide continuous, noninvasive blood pressure monitoring.

FIG. 5

EP 4 555 939 A2

## Description

### CROSS-REFERENCE

This application claims the benefit of U.S. Provisional Application No. 62/966,927, filed January 28, 2020, which application is incorporated herein by reference.

### BACKGROUND

Blood pressure is an essential vital sign routinely used to manage patient care. Methods of blood pressure measurement are typically cumbersome and bulky. Typical methods have several limitations, including susceptibility to ambient noise, patient discomfort, and inability to obtain a continuous blood pressure measurement. An alternative is invasive blood pressure measurement. While this provides much higher quality data than an external cuff its invasive nature also produces much higher risks, including infection, hemorrhage, or ischemia. Alternative non-invasive modalities for measuring blood pressure are highly desirable, particularly as hypertension has become an increasingly prevalent medical issue in both the United States and the rest of the world.

### SUMMARY

Provided herein are embodiments of a blood pressure measurement apparatus comprising: a first transducer configured to direct sound waves to a blood vessel; and a second transducer configured to direct ultrasonic waves to the blood vessel, receive ultrasonic waves reflected by echogenic boundaries of the blood vessel, and measure a radius and wall thickness of a cross section of the blood vessel. The first transducer may be an electroacoustic transducer and/or the second transducer is a piezoelectric ultrasonic transducer. The apparatus may further comprise an audio signal generator coupled to the first transducer. In some embodiments, the audio signal generator comprises at least variable resistor that adjusts a frequency of the sound waves. The variable resistor may be a potentiometer. In some embodiments, the audio signal generator displays the frequency of the sound waves. The second transducer may monitor a vibration of the cross-section of the blood vessel. In some embodiments, the second transducer monitors a vibration of the cross-section of the blood vessel, and wherein the piezoelectric ultrasonic transducer records the frequency of the vibration of the cross-section and determines a resonant frequency thereof. The resonant frequency may be determined when the vibration of the cross-section of the blood vessel is at a maximum. In some embodiments the frequency of the sound waves is varied over a range of 1 Hz to 3000 Hz. In some embodiments, the frequency of the sound waves is varied over a range of 670 Hz to 2300 Hz. The electroacoustic transducer may be an audio speaker, and the audio speaker may be a tweeter. The blood vessel may be an artery or a vein.

Further provided herein are embodiments of a method of measuring blood pressure comprising: determining a radius and thickness of a cross section of a blood vessel; directing sound waves to the blood vessel; varying a frequency of the sound waves; detecting a maximum resonance of the cross-section of the blood vessel to determine a resonant frequency of the blood vessel; and based on the determined resonant frequency, calculating a blood pressure in the blood vessel. Varying the frequency of the sound waves may comprise varying the frequency of the sound waves through a range of 1 Hz to 3000 Hz. In some embodiments, varying the frequency of the sound waves comprises varying the frequency of the sound waves through a range of 670 Hz to 2300 Hz. The resonant frequency may be determined by use of a piezoelectric ultrasonic transducer for detecting the maximum resonance. The piezoelectric ultrasonic transducer may comprise a sampling rate of at least 3 kHz. In some embodiments, determining the radius and thickness of the blood vessel comprises directing ultrasonic waves to the blood vessel and receiving reflected ultrasonic waves reflected from echogenic boundaries of the blood vessel. In some embodiments, the method may further comprise measuring a doppler shift of the reflected ultrasonic waves and calculating a wave velocity of the blood vessel. The doppler shift may be measured by a piezoelectric ultrasonic transducer. The blood vessel may be an artery or a vein.

Provided herein are embodiments of a blood pressure measurement device, comprising: a first transducer configured to emit multiple soundwaves having multiple frequencies, the soundwaves configured to cause a blood vessel of a subject to vibrate; a second transducer configured to capture one or more ultrasound images of the blood vessel; and a processing device configured to: determine, based on the one or more captured ultrasound images, a resonant frequency of the blood vessel; and calculate, based on a wall thickness of the blood vessel, a radius or diameter of the blood vessel, and the determined resonant frequency, a blood pressure of the blood vessel or the subject.

In some embodiments, the one or more captured ultrasound images are used to measure the wall thickness of the blood vessel and the radius or diameter of the blood vessel. In some embodiments, the one or more captured ultrasound images comprise multiple ultrasound images, wherein determining the resonant frequency of the blood vessel, comprises: determining, based on the multiple ultrasound images, a frequency of the multiple frequencies that maximized a vibration of the blood vessel; and selecting the frequency as the resonant frequency. In some embodiments, the device further comprises: an audio signal generator electrically coupled to the first transducer, the audio signal generator configured to adjust a frequency of sound waves emitted by the first transducer. In some embodiments, the audio signal generator comprises at least one variable resistor that adjusts the frequency of sound waves emitted by the first transducer. In some embodiments, each of the frequencies is between 1 Hz and 3000 Hz. In some embodiments, each of the frequencies is between 670 Hz and 2300 Hz.

In some embodiments, the blood vessel is a carotid artery of the subject. In some embodiments, wherein the first transducer is an audio speaker. In some embodiments, the device further comprises: a substrate, wherein the substrate comprises an adhesive surface for adhering to the subject's skin, wherein the first transducer and the second transducer are incorporated in the substrate. In some embodiments, the substrate is adhered proximal to the blood vessel. In some embodiments, the blood vessel is a carotid artery. In some embodiments, the substrate comprises an alignment line. In some embodiments, the substrate comprises a transparent window.

In some embodiments, the device further comprises a third transducer configured to capture a second set of one or more ultrasound images of the blood vessel. In some embodiments, the second and third transducers each have a respective resonant frequency, and wherein the second transducer comprises a frequency response that partially overlaps with a frequency response of the third transducer; and the processing device processes measurements made by the first and second transducers. In some embodiments, the processing of measurements made by the first and second transducers comprises normalizing the first frequency response and the second frequency response.

In some embodiments, the device further comprises a fourth transducer configured to capture a third set of one or more ultrasound images of the blood vessel. In some embodiments, the device further comprises: a substrate, wherein the substrate comprises an adhesive surface for adhering to the subject's skin, wherein the first transducer, the second transducer, and the third transducer are incorporated in the substrate. In some embodiments, the substrate is adhered proximal to the blood vessel. In some embodiments, the blood vessel is a carotid artery. In some embodiments, the substrate comprises an alignment line. In some embodiments, the substrate further comprises a transparent window.

In some embodiments, the device further comprises a fourth transducer configured to capture a third set of one or more ultrasound images of the blood vessel. In some embodiments, the fourth transducer comprises a frequency response that partially overlaps with the frequency response of the second transducer. In some embodiments, the fourth transducer comprises a frequency response that partially overlaps with the frequency response of the third transducer. In some embodiments, the fourth transducer comprises a frequency response that partially overlaps with the frequency response of the second and third transducers.

In some embodiments, the device further comprises: a substrate, wherein the substrate comprises an adhesive surface for adhering to the subject's skin, wherein the first transducer, the second transducer, and the third transducer are incorporated in the substrate. In some embodiments, the substrate is adhered proximal to the blood vessel. In some embodiments, the blood vessel is a carotid artery. In some embodiments, wherein the substrate comprises an alignment line. In some embodiments, the substrate further comprises a transparent window.

In some embodiments, the device further comprises a third transducer configured to capture a second set of one or more ultrasound images of the blood vessel. In some embodiments, the second and third transducers each have a respective resonant frequency, and wherein the second transducer comprises a frequency response that partially overlaps with a frequency response of the third transducer; and the processing device processes measurements made by the first and second transducers. In some embodiments, the processing of measurements made by the first and second transducers comprises normalizing the first frequency response and the second frequency response.

In some embodiments, the device further comprises a fourth transducer configured to capture a third set of one or more ultrasound images of the blood vessel. In some embodiments, the device further comprises a fourth transducer configured to capture a third set of one or more ultrasound images of the blood vessel. In some embodiments, the fourth transducer comprises a frequency response that partially overlaps with the frequency response of the second transducer. In some embodiments, the fourth transducer comprises a frequency response that partially overlaps with the frequency response of the third transducer. In some embodiments, the fourth transducer comprises a frequency response that partially overlaps with the frequency response of both the second and third transducers.

According to some embodiments, provided herein is a non-transitory computer-readable storage medium storing instructions executable by a processor, wherein execution of the instructions causes a blood pressure measurement device to perform operations comprising: emitting, using a first transducer in proximity to a blood vessel of a subject, multiple soundwaves having multiple frequencies, the soundwaves causing a blood vessel of a subject to vibrate; determining, based on a vibrational response of the blood vessel to the multiple soundwaves, a resonant frequency of the blood vessel; determining, using a second transducer that emits ultrasonic waves, a wall thickness and a radius or diameter of the blood vessel; and calculating, based on the resonant frequency, the wall thickness of the blood vessel, and the radius or diameter of the blood vessel, a blood pressure of the subject.

In some embodiments, the operations further comprise: capturing, using the second transducer, multiple ultrasound images of the blood vessel when it vibrates in response to the soundwaves, wherein determining the resonant frequency of the blood vessel comprises: determining the resonant frequency of the blood vessel from the ultrasound images. In some embodiments, determining the wall thickness and the radius of the blood vessel, comprises directing, using the second transducer, ultrasonic waves to the blood vessel; and receiving, using the second transducer, ultrasonic waves reflected from echogenic boundaries of the blood vessel. In some embodiments, after calculating the blood pressure, the operations further comprise: determining, using the first transducer and second transducer, an updated radius of the blood vessel and an updated velocity of blood flowing through the blood vessel; and calculating, based on the updated radius and the updated velocity, an updated blood pressure.

In some embodiments, the multiple frequencies of the multiple soundwaves are between 1 Hz and 3000 Hz. In some embodiments, the multiple frequencies of the multiple soundwaves are between 670 Hz and 2300 Hz. In some embodiments, the first transducer is an audio speaker.

In some embodiments, the operations further comprise capturing, using a third transducer, a first set of ultrasound images of the blood vessel. In some embodiments, the operations further comprise capturing, using the second transducer, a second set of ultrasound images of the blood vessel. In some embodiments, the operations further comprise normalizing the second set ultrasound images by the first set of ultrasound images.

In some embodiments, the operations further comprise capturing, using a fourth transducer, a third set of ultrasound images of the blood vessel. In some embodiments, the operations further comprise normalizing the second set ultrasound images by the first set of ultrasound images. In some embodiments, the operations further comprise normalizing the second set ultrasound images by the third set of ultrasound images.

According to some embodiments, provided herein is a method, comprising: emitting, using a first transducer in proximity to a blood vessel of a subject, multiple soundwaves having multiple frequencies, the soundwaves causing a blood vessel of a subject to vibrate; determining, based on the vibrational response of the blood vessel to the soundwaves, a resonant frequency of the blood vessel; determining, using a second transducer that emits ultrasonic waves, a wall thickness and a radius or diameter of the blood vessel; and calculating, based on the resonant frequency, the wall thickness of the blood vessel, and the radius or diameter of the blood vessel, a blood pressure of the subject.

In some embodiments, the method further comprises: capturing, using the second transducer, multiple ultrasound images of the blood vessel when it vibrates in response to the soundwaves, wherein determining the resonant frequency of the blood vessel comprises: determining the resonant frequency of the blood vessel from the ultrasound images. In some embodiments, determining the wall thickness and the radius of the blood vessel, comprises directing, using the second transducer, ultrasonic waves to the blood vessel; and receiving, using the second transducer, ultrasonic waves reflected from echogenic boundaries of the blood vessel.

In some embodiments, calculating the blood pressure, the method further comprises: determining, using the first transducer and second transducer, an updated radius of the blood vessel and an updated velocity of blood flowing through the blood vessel; and calculating, based on the updated radius and the updated velocity, an updated blood pressure. In some embodiments, each of the frequencies is between 670 Hz and 2300 Hz.

In some embodiments, the method further comprises capturing, using a third transducer, a second set of multiple ultrasound images of the blood vessel when it vibrates in response to the soundwaves. In some embodiments, the method further comprises normalizing the multiple ultrasound images of the blood vessel captured by the second transducer with the second set of multiple ultrasound images of the blood vessel captured by the third transducer.

In some embodiments, the method further comprises capturing, using a fourth transducer, a third set of multiple ultrasound images of the blood vessel when it vibrates in response to the soundwaves. In some embodiments, the method further comprises normalizing the multiple ultrasound images of the blood vessel captured by the second transducer with the second set of multiple ultrasound images of the blood vessel captured by the third transducer. In some embodiments, the method further comprises normalizing the multiple ultrasound images of the blood vessel captured by the second transducer with the third set of multiple ultrasound images of the blood vessel captured by the fourth transducer.

According to some embodiments, provided herein is a blood pressure measurement apparatus comprising: a first transducer configured to direct sound waves to a blood vessel; and a second transducer configured to direct ultrasonic waves to the blood vessel, receive ultrasonic waves reflected by echogenic boundaries of the blood vessel, measure a or diameter radius of a cross-section of the blood vessel and measure a wall thickness of the cross section of the blood vessel.

In some embodiments, the first transducer is an electroacoustic transducer and the second transducer is a piezoelectric ultrasonic transducer. In some embodiments, the apparatus further comprises an audio signal generator coupled to the first transducer. In some embodiments, the audio signal generator is configured to vary a frequency of the sound waves. In some embodiments, the apparatus further comprises a display. In some embodiments, the display shows the frequency of the sound waves.

In some embodiments, the second transducer monitors a vibration of the cross-section of the blood vessel. In some embodiments, the second transducer monitors a vibration of the cross-section of the blood vessel, and wherein the second transducer records the frequency of the vibration of the cross-section and determines a resonant frequency thereof. In some embodiments, wherein the resonant frequency is determined when the vibration of the cross-section of the blood vessel is at a maximum

In some embodiments, a frequency of the sound waves is varied over a range of 1 Hz to 3000 Hz. In some embodiments, a frequency of the sound waves is varied over a range of 670 Hz to 2300 Hz. In some embodiments, the electroacoustic transducer is an audio speaker. In some embodiments, the audio speaker is a tweeter.

In some embodiments, the blood vessel is an artery or a vein. In some embodiments, the first and second transducers are coupled to a substrate, and wherein the substrate comprises an adhesive backing. In some embodiments, the substrate is adhered proximal to the blood vessel. In some embodiments, the blood vessel is a carotid artery. In some embodiments, the substrate comprises an alignment line. In some embodiments, the substrate comprises an alignment line and a transparent window.

According to some embodiments, provided herein is a method of measuring blood pressure comprising: determining a radius and wall thickness of a cross section of a blood vessel; directing sound waves to the blood vessel; varying a frequency of the sound waves; detecting a maximum resonance of the cross-section of the blood vessel to determine a resonant frequency of the blood vessel; and calculating, based on the determined resonant frequency, radius, and wall thickness of the blood, a blood pressure in the blood vessel.

In some embodiments, varying the frequency of the sound waves comprises varying the frequency of the sound waves through a range of 1 Hz to 3000 Hz. In some embodiments, varying the frequency of the sound waves comprises varying the frequency of the sound waves through a range of 670 Hz to 2300 Hz.

In some embodiments, the resonant frequency is determined by use of a piezoelectric ultrasonic transducer for detecting the maximum resonance. In some embodiments, the piezoelectric ultrasonic transducer comprises a sampling rate of at least 3 kHz. In some embodiments, the step of determining the radius and wall thickness of the blood vessel comprises directing ultrasonic waves to the blood vessel and receiving reflected ultrasonic waves reflected from echogenic boundaries of the blood vessel. In some embodiments, the method further comprises measuring a doppler shift of the reflected ultrasonic waves and calculating a wave velocity of the blood vessel. In some embodiments, the doppler shift is measured by a piezoelectric ultrasonic transducer.

In some embodiments, the blood vessel is an artery or a vein. In some embodiments, the method further comprises directing a first set of transmitted ultrasound waves toward the blood vessel and capturing a first set of reflected ultrasound waves from the blood vessel. In some embodiments, the steps of directing a first set of transmitted ultrasound waves toward the blood vessel and capturing a first set of reflected ultrasound waves from the blood vessel are conducted by a single ultrasound transducer.

In some embodiments, the method further comprises directing, with a first ultrasound transducer, a first set of transmitted ultrasound waves toward the blood vessel and capturing, with a second ultrasound transducer, a first set of reflected ultrasound waves from the blood vessel. In some embodiments, the method further comprises directing, with a first ultrasound transducer, a first set of transmitted ultrasound waves toward the blood vessel and capturing, with the first ultrasound transducer and a second ultrasound, a first set of reflected ultrasound waves from the blood vessel. In some embodiments, the method further comprises directing, with a first ultrasound transducer, a first set of transmitted ultrasound signal toward the blood vessel and capturing, with the first ultrasound transducer, a first reflected ultrasound signal from the blood vessel.

In some embodiments, the method further comprises capturing, with a second ultrasound transducer, a second reflected ultrasound signal from the blood vessel. In some embodiments, the method further comprises normalizing the first reflected ultrasound signal with the second reflected ultrasound signal.

In some embodiments, the method further comprises capturing, with a third ultrasound transducer, a third reflected ultrasound signal from the blood vessel. In some embodiments, the method further comprises normalizing the first reflected ultrasound signal with the third reflected ultrasound signal. In some embodiments, the blood vessel is carotid artery.

According to some embodiments, provided herein is a system for transmitting and receiving ultrasound signals comprising: a software-defined radio comprising one or more outputs and one or more inputs; an ultrasound signal processing circuit electrically coupled to the software-defined radio; and one or more ultrasound transducers electrically coupled to the ultrasound signal processing circuit, wherein the ultrasound signal processing circuit processes one or more ultrasound transmission signals from the one or more outputs of the software-defined radio and transmits processed ultrasound transmission signals to the one or more ultrasound transducers to produce ultrasound waves, and wherein the signal processing unit processes one or more received ultrasound signals from the one or more ultrasound transducers and transmits processed received ultrasound signals to the one or more inputs of the software-defined radio.

In some embodiments, the ultrasound processing circuit comprises at least one high voltage amplifier to amplify the one or more ultrasound transmission signals from the one or more outputs of the software-defined radio. In some embodiments, the ultrasound processing circuit comprises at least one variable gain amplifier to amplify the one or more received ultrasound signals from the one or more ultrasound transducers.

In some embodiments, the ultrasound processing circuit comprises at least one variable gain amplifier to amplify the one or more received ultrasound signals from the one or more ultrasound transducers. In some embodiments, the outputs of the software-defined radio comprise a gain ramp and a transmission pulse. In some embodiments, the ultrasound processing circuit comprises a high voltage amplifier to amplify the transmission pulse.

In some embodiments, the ultrasound processing circuit comprises a variable gain amplifier to amplify the one or more received ultrasound signals from the one or more ultrasound transducers based on the gain ramp transmitted by the software-defined radio. In some embodiments, a rate of amplification of the ramp signal corresponds to the time since an initial pulse of the ramp signal was emitted by the software-defined radio.

In some embodiments, the ultrasound transducers comprise a first pixel group and a second pixel group. In some embodiments, the first pixel group and the second pixel group each comprise 16 pixels. In some embodiments, the first pixel group is configured to receive the processed ultrasound transmission signals. In some embodiments, only the first pixel group receives the processed ultrasound transmission signals.

In some embodiments, the one or more received ultrasound signals are received transmitted to the ultrasound processing circuit by the first pixel group and the second pixel group. In some embodiments, the variable gain amplifier is a low noise, single-ended, linear, general purpose variable gain amplifier.

In some embodiments, the ultrasound transducers comprise a first pixel group and a second pixel group. In some embodiments, the first pixel group is configured to receive the processed ultrasound transmission signals. In some embodiments, the first pixel group receives the processed ultrasound transmission signals.

In some embodiments, the system further comprises a computing device connected to the software-defined radio, such that the computing device controls the one or more outputs of the software-defined radio. In some embodiments, the computing device comprises a display, wherein the display displays data received by the one or more inputs of the software-defined radio. In some embodiments, the display displays one or more ultrasonic images obtained by the one or more ultrasound transducers.

In some embodiments, the system further comprises a computing device connected to the software-defined radio, such that the computing device controls the one or more outputs of the software-defined radio. In some embodiments, the computing device comprises a display, wherein the display displays data received by the one or more inputs of the software-defined radio.

The system of paragraph 123, wherein the display displays one or more ultrasonic images obtained by the one or more ultrasound transducers.

According to some embodiments, provided herein is a method of conditioning one or more ultrasound signals comprising: receiving a first transmission signal from a software-defined radio; amplifying the first transmission signal, thereby forming an amplified transmission signal; forwarding, with a first multiplexer, the amplified transmission signal to one or more ultrasound transducers; receiving, with the first multiplexer, one or more received ultrasound signals from the one or more ultrasound transducers; and amplifying the one or more received ultrasound signals, thereby forming one or more amplified received ultrasound signals.

In some embodiments, amplification of the one or more received ultrasound signals is based on a gain ramp transmitted by the software-defined radio. In some embodiments, the amplification is time dependent. In some embodiments, amplification of the one or more received ultrasound signals is carried out by one or more low noise, single-ended, linear, general purpose variable gain amplifiers. In some embodiments, amplification of the one or more received ultrasound signals is carried out by one or more low noise, single-ended, linear, general purpose variable gain amplifiers.

In some embodiments, the method further comprises emitting one or more ultrasound waves toward at least one object, wherein the at least one object reflects the one or more ultrasound waves, and wherein the reflected ultrasound waves are detected by the one or more ultrasound transducers and form the one or more received ultrasound signals.

In some embodiments, amplification of the one or more received ultrasound signals is based on a gain ramp transmitted by the software-defined radio. In some embodiments, the amplification is time dependent. In some embodiments, amplification of the one or more received ultrasound signals is carried out by one or more low noise, single-ended, linear, general purpose variable gain amplifiers.

In some embodiments, the at least one object is a blood vessel of a subject. In some embodiments, the blood vessel is a carotid artery.

**BRIEF DESCRIPTION OF THE DRAWINGS**

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:

FIG. 1 depicts forces acting upon an arterial membrane, according to some embodiments;

FIG. 2 depicts vibration of an arterial wall at a resonance frequency, according to some embodiments;

FIG. 3 is a block diagram illustrating some components of a continuous blood pressure measurement device, in accordance with some embodiments of the disclosure;

FIG. 4 depicts echogenic boundaries of an arterial wall, according to some embodiments;

FIG. 5 illustrates an adhesive patch including an integrated continuous blood pressure measurement device, according to some embodiments;

FIG. 6 is an operational flow diagram illustrating an example method for measuring blood pressure of a subject, according to some embodiments;

FIGS. 7A-7B depict frequency responses of three ultrasound sensors of a continuous ultrasound measurement device, according to some embodiments;

FIGS. 8A-8D depict a technique for cross-correlation in an ultrasound measurement device;

FIG. 9 depicts a block diagram illustrating an example system, according to some embodiments;

FIG. 10 illustrates a computer system that is programmed or otherwise configured to implement methods provided herein, according to some embodiments;

FIG. 11 illustrates a method of forming ultrasonic images from a plurality of ultrasonic transducers, according to some embodiments; and

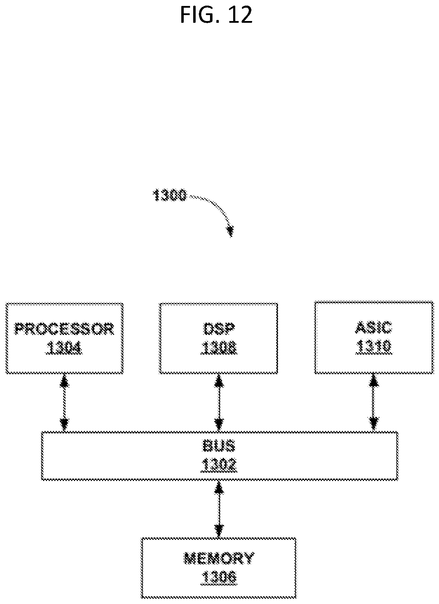

FIG. 12 illustrates an example chip set that can be utilized in implementing architectures and methods in accordance with various embodiments of the disclosure.

## DETAILED DESCRIPTION

Provided herein is a device which will enable continuous, non-invasive blood pressure measurement. In some embodiments, the device comprises one or more ultrasound transducers. In some embodiments, the device presented herein removes the need for a calibration step and provides a continuous, non-invasive blood pressure monitoring capability in an inexpensive and easy-to-use form.

According to some embodiments, the technology disclosed herein is directed to a blood pressure measurement apparatus that enables continuous, non-invasive blood pressure measurement using sound and ultrasound transducers. In some embodiments, an electroacoustic transducer is used to generate an audio signal that stimulates vibrations in an artery. By varying the frequency of the acoustic signal, a resonant frequency at which the artery vibrates most strongly may be determined. This resonant frequency may be combined measurements of the radius of the artery to calculate an absolute blood pressure. In some embodiments, the radius of the artery is measured by one or more of the ultrasound transducers utilizing ultrasound imaging methods. Methods of measuring blood pressure utilizing acoustic stimulation may eliminate the need for a separate calibration step and blood pressure monitoring may be provided in an inexpensive and easy-to-use form.

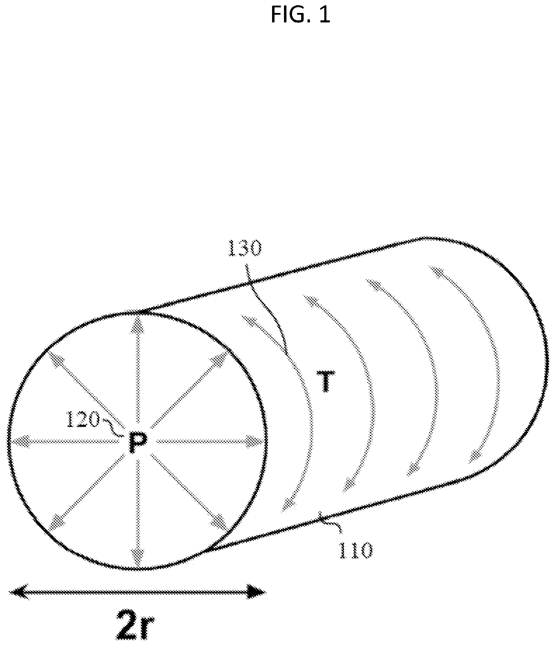

To understand the principles underlying the technology described herein, it is instructive to consider the forces underlying blood flow through an artery. FIG. 1 depicts an arterial wall 110, which may be represented as a tube composed of elastic material. The dynamics of arterial wall 110 are dictated by the balance between blood pressure 120 pushing the wall out and tensile force 130 holding the wall together. Laplace's law states that for fluid flowing through a cylindrical elastic membrane with constant radius R, the fluid pressure P and the tensile force in the membrane $T$ are related by the equation $T = RP$.

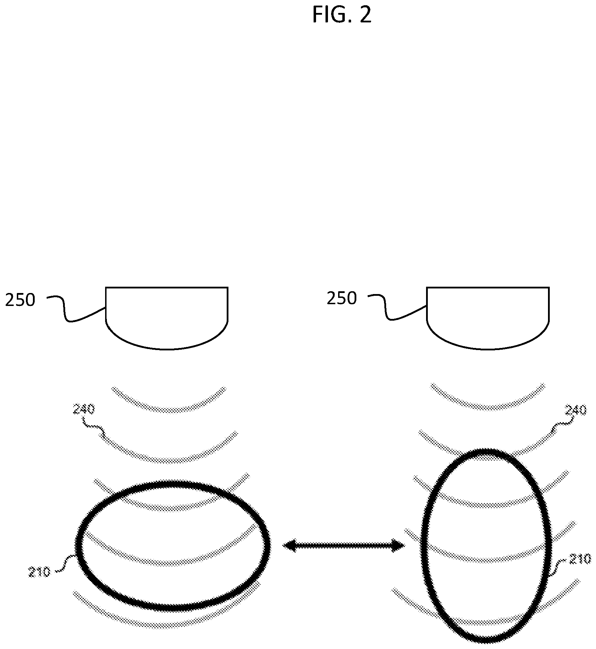

The radius of the artery may be measured using one or more ultrasound devices. As such, the remaining task for determining the fluid pressure P is measuring the tensile force in the wall of the artery. In some embodiments, the tensile force may be determined by measuring how the arterial wall responds to an impulse from an acoustic stimulus. FIG. 2 depicts this process. In some embodiments, an acoustic stimulus 240, produced by an electroacoustic transducer 250, is used to induce measurable perturbations in the arterial wall 210. This method may utilize an acoustic stimulus which is emitted at an acoustic intensity well below the acoustic intensity limits specified by FDA guidelines. By focusing on vibrational modes around the circumference of the artery, and particularly, the lowest-energy vibration which can be excited around the circumference, the tensile force may be estimated as follows.

The lowest-energy vibration has a wavelength of $\lambda = \pi R$. The mechanical properties of the arterial wall also dictate a speed, $v_w$, at which vibrations travel along the circumference of the wall. The wave speed and wavelength quantities combine to produce a resonant frequency $f = v_w/\lambda$. If an external stimulus (e.g. a sounds wave) is applied with frequency $f$, the wall will vibrate very strongly, but if the frequency of the stimulus is varied away from $f$, the amplitude of the vibration will decrease. Thus, $f$ may be determined by applying an acoustic stimulus across a frequency range and finding the frequency at which the artery vibrates most strongly. This, in turn, will tell us the wave speed $v_w = \lambda f = \pi R f$. In an elastic material under tension, with volumetric density $\rho$ and thickness $h$, this wave velocity is related to the tensile force via the equation $T = \rho h v_w^2$. The volumetric density of the arterial wall is nearly constant across patients, and the wall thickness may be measured from an ultrasound image in the same manner as R. Using this information, pressure may be determined based on Equation (1):

$$P = T/R = \rho h v_w{}^2/R = \pi^2 \rho h R f^2 \qquad (1)$$

Thus, measuring $h$ and R via standard ultrasound imaging and measuring *f* by determining the resonance point by the artery's vibrational response will allow calculation of a blood pressure.

## I. FEASIBILITY ANALYSIS.

To illustrate the feasibility of the continuous blood pressure measurement techniques described herein, the following factors may be considered. First, to illustrate the practically of this blood pressure measurement method, it may be ensured that the resonant frequency, *f*, will take on a reasonable value in the typical patient. In a common carotid artery, the measurement quantities typically have values of about 100 mmHg for *P*, 2.5 mm for *R*, 0.3 mm for *h*, and 1 g/mL for *p*. Inserting these values into Equation (1) yields a predicted resonant frequency of approximately 1.3 kHz. Thus, in order to detect this vibration, sampling may need to be conducted at least ~3 kHz in order to surpass the Nyquist rate. Currently available ultrasound instruments may produce imaging rates of at least 8 kHz, far surpassing the minimum sampling rate. 1.3 kHz is also far higher than the frequency of heartbeats, which in human patients does not exceed 5 Hz even under extreme conditions. Because resonant vibrations occur at a much higher frequency than the rate of change in pressure from the heartbeat, blood pressure (and thus the tensile force) may be assumed as constant over the duration of each vibrational cycle, substantially simplifying analysis. Furthermore, 1.3 kHz falls comfortably in the range of existing commercial speakers, so no specialized hardware is required to produce a tone with sufficient amplitude and frequency to stimulate arterial vibration.

Second, another measure of feasibility is the frequency range over which the electroacoustic transducer may need to operate. Under extreme conditions, a patient's blood pressure may be as low as 25 mmHg or as high as 300 mmHg. With the arterial dimensions given above, this corresponds to a resonant frequency range of 670 Hz to 2.3 kHz, still well within the acoustic range produced by commercial speakers.

Third, the level of frequency resolution required to obtain a useful level of precision in pressure measurements may also be considered. In order to be comparable to the current clinical gold standard of continuous blood pressure measurement, a blood pressure measurement device should be able to measure pressure to a precision of at least $\pm 5$ mmHg. With the arterial dimensions given above, a blood pressure swing of this magnitude would result in a change in the resonant frequency of $\pm 34$ Hz, or roughly 2.5%. This level of precision can also be readily obtained from commercially available speakers.

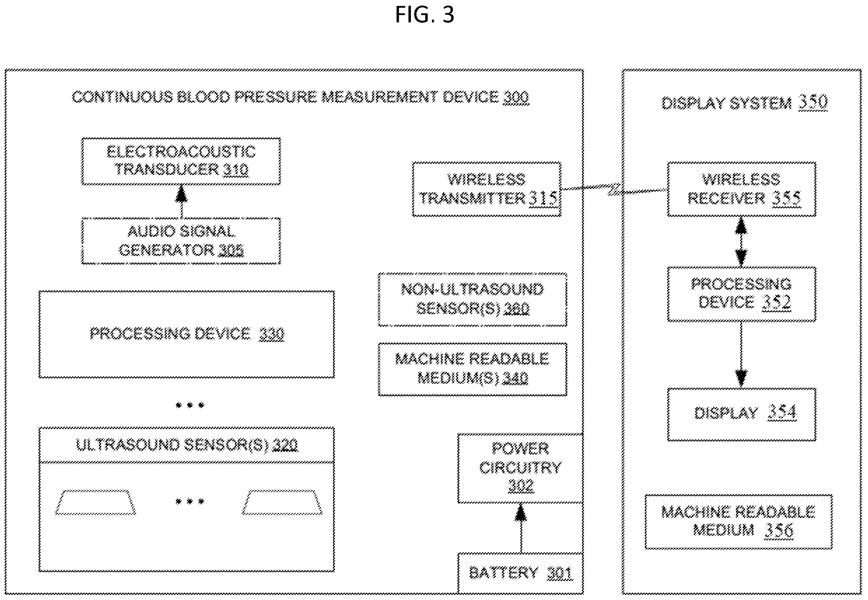

FIG. 3 is a block diagram illustrating some components of a continuous blood pressure measurement device 300, in accordance with some embodiments of the disclosure. As depicted, device 300 includes an audio signal generator 305, an electroacoustic transducer 310, at least one ultrasound sensor 320, at least one processing device 330, at least one machine readable medium 340, a wireless transmitter 315, and, optionally, one or more non-ultrasound sensor(s) 360. The electrical components of device 300 may be powered by a battery 301 that connects to power circuitry 302 for distributing power. The battery 301 may be rechargeable (e.g., via a USB port and/or an AC/DC converter). Although a battery 301 is shown in this example, it should be appreciated that any suitable battery or power supply technologies may be used to power the components of device 300. For example, lithium-ion batteries, cell batteries, piezo or vibration energy harvesters, photovoltaic cells, AC/DC sources, or other like devices can be used.

Electroacoustic transducer 310 may be implemented as an audio speaker that can output sound waves within a suitable range of frequencies (e.g., to find a resonant frequency of a blood vessel). For example, the speaker may be implemented as a bass speaker, a mid-range speaker, and/or a treble speaker (e.g., tweeter). In some embodiments, a combination of speakers may be used to output sound within a suitable range of frequencies. An audio signal generator 305 that adjusts a frequency of sound waves may be coupled to transducer 310. In some embodiments, the audio signal generator comprises at least a variable resistor that adjusts a frequency of the sound waves. The variable resistor may be a potentiometer. In some embodiments, the blood pressure measurement device 300 may display the frequency of the sound waves. In some embodiments, the audio signal generator 305 is a component of a processing device 330.

In some embodiments, the sound waves output by electroacoustic transducer 310 may be configured to be varied over a range of frequencies from about 100 Hz to about 3,500 Hz. In an embodiment, the range of frequencies the electroacoustic transducer 310 is varied over is about 100 Hz to about 500 Hz, about 100 Hz to about 1,000 Hz, about 100 Hz to about 1,500 Hz, about 100 Hz to about 2,000 Hz, about 100 Hz to about 2,500 Hz, about 100 Hz to about 3,000 Hz, about 100 Hz to about 3,500 Hz, about 500 Hz to about 1,000 Hz, about 500 Hz to about 1,500 Hz, about 500 Hz to about 2,000 Hz, about 500 Hz to about 2,500 Hz, about 500 Hz to about 3,000 Hz, about 500 Hz to about 3,500 Hz, about 1,000 Hz to about 1,500 Hz, about 1,000 Hz to about 2,000 Hz, about 1,000 Hz to about 2,500 Hz, about 1,000 Hz to about 3,000 Hz, about 1,000 Hz to about 3,500 Hz, about 1,500 Hz to about 2,000 Hz, about 1,500 Hz to about 2,500 Hz, about 1,500 Hz to about 3,000 Hz, about 1,500 Hz to about 3,500 Hz, about 2,000 Hz to about 2,500 Hz, about 2,000 Hz to about 3,000 Hz, about 2,000 Hz to about 3,500 Hz, about 2,500 Hz to about 3,000 Hz, about 2,500 Hz to about 3,500 Hz, or about 3,000 Hz to about 3,500 Hz. In some embodiments, the range of frequencies the electroacoustic transducer 310 is varied over is about 100 Hz, about 500 Hz, about 1,000 Hz, about 1,500 Hz, about 2,000 Hz, about 2,500 Hz, about 3,000 Hz, or about 3,500 Hz. In some embodiments, the range of frequencies the electroacoustic transducer 310 is varied over is at least about 100 Hz, about 500 Hz, about 1,000 Hz, about 1,500 Hz, about 2,000 Hz, about 2,500 Hz, or about 3,000 Hz. In some embodiments, the range of frequencies the electroacoustic transducer 310 is varied over is at most about 500 Hz, about 1,000 Hz, about 1,500 Hz, about 2,000 Hz, about 2,500 Hz, about 3,000 Hz, or about 3,500 Hz.

] In some embodiments, audio signal generator 305 may be configured to adjust the frequency of sound waves output by transducer 310 in suitable increments to search for the resonant frequency of vibration at a sufficient accuracy. For example, the frequency may be adjusted in increments between 1 Hz and 500 Hz such as 1 Hz, 5 Hz, 10 Hz, 25 Hz, 50 Hz, 100 Hz, 200 Hz, 500 Hz, etc. The accuracy of the estimated resonant frequency may be improved by decreasing the size of each increment. Conversely, the time it takes to determine the resonant frequency may be improved by increasing the size of each increment.

The one or more ultrasound sensors 320 (individually referred to as "an ultrasound sensor 320") are configured to collect imaging data of a subject (e.g., blood vessel a subject). The imaging data may be used to measure the wall thickness of a blood vessel and the radius of the blood vessel. The blood vessel may be an artery or a vein. Additionally, the imaging data may be used to determine the resonant frequency of vibration of the blood vessel, which vibrates in response to the sound waves output by electroacoustic transducer 310. Each ultrasound sensor 320 includes a transducer configured to convert electricity to ultrasound and vice versa. For example, the transducer may be a piezoelectric transducer that oscillates and produces an ultrasonic pulse when an AC voltage is applied. Alternatively, the transducer may be a capacitive transducer that produces ultrasonic soundwaves using electrostatic fields between a conductive diaphragm and a backing plate. Ultrasound soundwaves may be a produced at a frequency greater than or equal to about 20 kilohertz (KHz). In some embodiments, the transducer of an ultrasound sensor 320 may produce ultrasound in a frequency anywhere between 2 megahertz (MHz) and 20 MHz When a reflected ultrasound signal (i.e., an "echo") is received by the transducer, an electrical signal may be generated and used by ultrasound sensor 120 to determine a distance to the imaged subject. In some embodiments, the transducer may produce ultrasound in a frequency between 7 MHz and 11 MHz

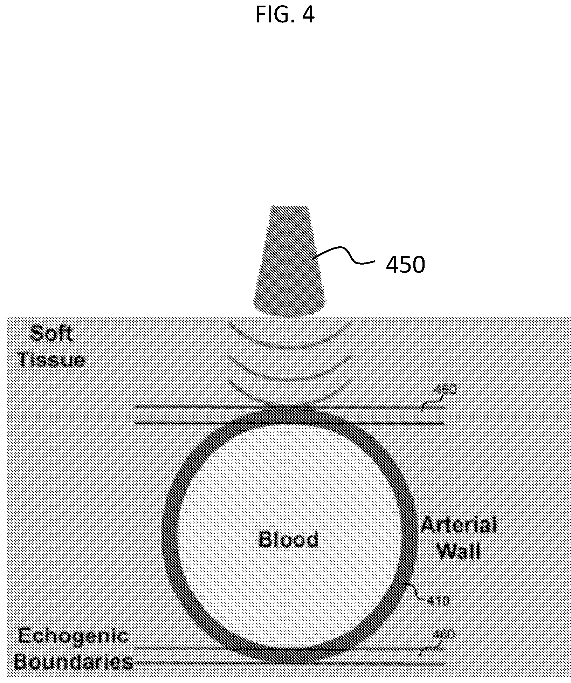

In some embodiments, ultrasonic measurement is performed using only a single piezoelectric element. Ultrasonic waves in the body are primarily reflected by sharp boundaries between regions of different densities. Many arteries, including the carotid, are embedded in soft tissue of relatively uniform density, so the only significant sources of echoes are the boundaries 460 formed by the inner and outer edges of the arterial wall 410 as depicted by FIG. 4. By measuring the delay between echoes received from these different boundaries, accurate determination of arterial radius and wall thickness from a single piezoelectric ultrasound transducer element 450 is achievable.

As depicted in FIG. 2, when an acoustic stimulus 240 is applied to an artery (e.g., using a tweeter), the resulting vibration will cause the arterial diameter, observed using ultrasound imaging, to oscillate sinusoidally as the axis perpendicular to the incident sound waves expands and contracts. If the power of the stimulus is kept constant this oscillation will have maximum amplitude when the stimulus is applied at a frequency matching the resonant frequency of the arterial wall 210. Thus, the resonant frequency may be determined by applying the acoustic stimulus at a range of different frequencies and determining, via ultrasound imaging, the point at which the observed amplitude of oscillation is at a maximum. With this setup, the velocity of the wall may also be determined independently of its position by measuring the Doppler shift of the returned echo. Since wall velocity will also be maximized at resonance, the velocity measurement will give an orthogonal means of determining the resonance point and thereby increase precision.

In a further embodiment, precision may be increased by increasing the number of ultrasonic piezoelectric elements. In some embodiments, four piezoelectric elements may be utilized. One of the piezoelectric elements could produce an ultrasonic signal while the other three listen for echoes, allowing for triangulation of echoes as they are received. This embodiment may allow for better exclusion of spurious echoes and thereby provide better isolation of echoes being produced by the arterial wall.

Ultrasound that enters tissue may be transmitted, attenuated or reflected. While higher frequency ultrasound may provide for a higher resolution signal, it may provide poor depth penetration of imaged tissue. Conversely, while lower frequency ultrasound may provide for a lower resolution signal, it may provide better depth penetration of imaged tissue. To overcome these limitations, some embodiments may use multiple ultrasound sensors 320, with each ultrasound sensor 320 having a transducer configured with a unique resonance frequency. Specifically, the ultrasound transducers may be selected such that their acoustic frequency responses are non-overlapping. By actuating the transducers in a time interleaved fashion, each sensor may be used to image a surface of a sample at a unique depth. Furthermore, each transducer may be simultaneously actuated with a combination of other transducers to generate higher order harmonics that allow for sub-pixel feature resolution. In some embodiments, ultrasound sensor transducers may be selected that are adjacent and partially overlapping in frequency response, allowing for the normalization of the measurement of any pair of sensors, thereby reducing systemic sources of noise and significantly increasing signal integrity. Additionally, the simultaneous interrogation of a surface with transducers with distinct resonance frequencies may be used to generate super-resolution ultrasound images. In some embodiments, the frequency overlap between sensors may be configured to be about 200 KHz or less. In some embodiments, the frequency overlap between sensors may be configured such that a frequency response range of one ultrasound sensor does not overlap with a resonant frequency of another ultrasound sensor, where the resonant frequency of the ultrasound sensor may refer to an operating frequency at which the transducer most efficiently converts electrical energy into mechanical energy.

Processing device 330 may be configured to control operation of the components of device 300, including audio signal generator 305 (which in some embodiments is a component of processing device 130), electroacoustic transducer 310, ultrasound sensor(s) 320, and non-ultrasound sensor(s) 360 (further discussed below). For example, processing device 330 may be configured to cause electroacoustic transducer 310 to emit sound at a specific frequency or range of frequencies. Additionally, processing device 330 may configured to cause ultrasound sensors 320 and/or non-ultrasound sensor(s) 360 to perform image acquisition. Furthermore, processing device 330 may receive, store (e.g., in machine readable medium 340), and/or process signal measurements received from ultrasound sensors 320 and/or non-ultrasound sensors 360. In some embodiments, processing device 330 may also be configured to use signal measurements received from ultrasound sensors 320 to continuously measure blood pressure of an artery or other blood vessel. The aforementioned methods may be applied by processing device 330 by executing instructions stored on a machine readable medium 340. In one embodiment, processing device 330 may be implemented as a single integrated circuit (IC) microcontroller that includes memory (e.g., machine readable medium 340) for storing program information and data.

As described above, continuous blood pressure measurement device 300 may accurately determine blood pressure solely from arterial radius, wall thickness, and resonant frequency. However, those three values are not the only information that may be determined. In some embodiments, in the course of measuring the vibrational response of the artery under a range of frequencies, additional information may be obtained, including: the strength and width of the resonance peak, the baseline excitation level at low frequencies, and/or the level of excitation of higher-energy vibrational modes. Changes in these parameters may also be observed over the course of a heartbeat as blood pressure and tension levels change. Ultrasound imaging of the artery may also yield information about the echogenicity of the arterial wall, which may provide insight into levels of calcification and plaque buildup. All the additional information may be used to refine calculations, attain better precision, and/or provide additional information about the patient's overall health.

In some embodiments, continuous blood pressure measurement device 300 may include one or more non-ultrasound sensors 360 to allow for multi-modal measurement of other health indicators besides blood pressure. For example, LED light sources and photodiode receivers may be integrated onto the wireless platform to measure blood oxygenation through pulse oximetry. Other example sensors that may be implemented include sensors for detecting body fluid status, ejection fraction of the heart, or other vital measurements may be integrated into the hardware/sensor package. In some embodiments, the measurements made using non-ultrasound sensors 360 may be correlated and normalized with ultrasound measurements used to calculate blood pressure. By virtue of using additional modalities besides ultrasound sensors 320 to measure blood pressure, the accuracy of device 300 may be improved.

In the example, of FIG. 3, continuous blood pressure measurement device 300 includes a wireless transmitter 315 (e.g., transceiver) that is configured to communicate ultrasound measurement data to wireless receiver 355 (e.g., transceiver) of display system 350. Depending on the ultrasound imaging application, the received ultrasound measurement data may be processed using processing device 352 of display system 355 (e.g., into a format appropriate for display) and/or displayed using display 354. Instructions for formatting may be stored on a machine readable medium 356. For example, the display 354 may be a component of a cardiac monitor, a mobile device (e.g., smartphone or head mounted display), or some other suitable display device. The display 354 may also display the frequency of sound waves electroacoustic transducer 310 is configured to emit (e.g., via audio signal generator 305). The wireless communication link between wireless transmitter 315 and wireless receiver 355 may be a radio frequency link such as a Bluetooth® or Bluetooth® low energy (LE) link, a Wi-Fi® link, a ZigBee link, or some other suitable wireless communication link. In some embodiments, data transfer between device 300 and display system 350 may be achieved using a wired transmitter or other suitable wired interface. For example, data may be transferred using a USB-C connector, a USB 2.x or 3.x connector, a micro-USB connector, a THUNDERBOLT connector, an Ethernet cable, etc.

In some embodiments, display 354 and/or the functions of display system 350 may be integrated into continuous blood pressure measurement device 300. In some embodiments, continuous blood pressure measurement device 300 may still retain transmitter 315 to communicate historical or current blood pressure measurement data to an external device such as a smartphone.

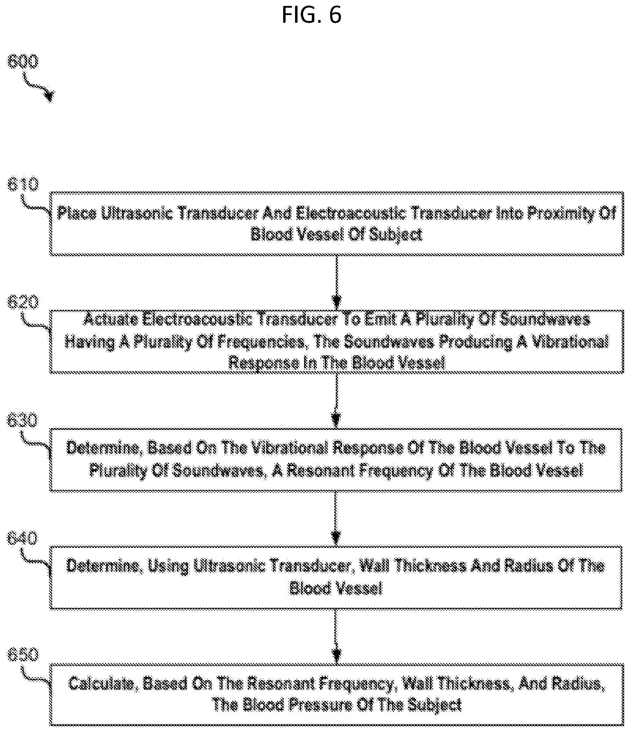

FIG. 6 is an operational flow diagram illustrating an example method 600 for measuring blood pressure of a subject, in accordance with some embodiments of the disclosure. In some embodiments, method 600 may be implemented using a continuous blood pressure measurement device 300 as described above.

At operation 610, an ultrasound transducer and electroacoustic transducer are brought into proximity of a blood vessel of the subject. For example, a continuous blood pressure measurement device 300 including an electroacoustic transducer 310 and ultrasound sensor 320 may be brought into proximity of an artery (e.g., carotid artery or brachial artery) of the patient. In some embodiments, a substrate 500 including device 300 may be aligned with the subject's artery and subsequently adhered. For example, the substrate 500 may be adhered to the patient's arm in proximity to the brachial artery. Alternatively, the substrate may be adhered to the patient's neck in proximity to the carotid artery. In some embodiments, the position of the ultrasound transducer and electroacoustic transducer (e.g., device 300 or substrate 500) may be adjusted to maximize the amplitude and/or signal-to-noise ratio of measurement signals.

At operation 620, the electroacoustic transducer is actuated to emit a plurality of soundwaves having a plurality of frequencies. For example, audio signal generator 305 may be configured to cause electroacoustic transducer 310 to emit sound waves at a plurality of different frequencies. The sound waves may produce a vibrational response in blood vessel (e.g., in a cross section of the blood vessel that the sound waves are directed to). The vibrational response may have an amplitude that varies depending on the frequency of the sound waves.

In some embodiments, the frequency of each of the sound waves may be between 1 Hz and 3000 Hz. In some embodiments, the frequency of each of the sound waves may be between 670 Hz and 2300 Hz. In some embodiments, the frequency of each of the soundwaves is about 300 Hz to about 3,000 Hz. In some embodiments, the frequency of each of the soundwaves about 1 Hz to about 3,000 Hz. In some embodiments, the frequency of each of the soundwaves about 1 Hz to about 300 Hz, about 1 Hz to about 500 Hz, about 1 Hz to about 750 Hz, about 1 Hz to about 1,000 Hz, about 1 Hz to about 1,500 Hz, about 1 Hz to about 2,000 Hz, about 1 Hz to about 2,500 Hz, about 1 Hz to about 3,000 Hz, about 300 Hz to about 500 Hz, about 300 Hz to about 750 Hz, about 300 Hz to about 1,000 Hz, about 300 Hz to about 1,500 Hz, about 300 Hz to about 2,000 Hz, about 300 Hz to about 2,500 Hz, about 300 Hz to about 3,000 Hz, about 500 Hz to about 750 Hz, about 500 Hz to about 1,000 Hz, about 500 Hz to about 1,500 Hz, about 500 Hz to about 2,000 Hz, about 500 Hz to about 2,500 Hz, about 500 Hz to about 3,000 Hz, about 750 Hz to about 1,000 Hz, about 750 Hz to about 1,500 Hz, about 750 Hz to about 2,000 Hz, about 750 Hz to about 2,500 Hz, about 750 Hz to about 3,000 Hz, about 1,000 Hz to about 1,500 Hz, about 1,000 Hz to about 2,000 Hz, about 1,000 Hz to about 2,500 Hz, about 1,000 Hz to about 3,000 Hz, about 1,500 Hz to about 2,000 Hz, about 1,500 Hz to about 2,500 Hz, about 1,500 Hz to about 3,000 Hz, about 2,000 Hz to about 2,500 Hz, about 2,000 Hz to about 3,000 Hz, or about 2,500 Hz to about 3,000 Hz. In some embodiments, the frequency of each of the soundwaves about 1 Hz, about 300 Hz, about 500 Hz, about 750 Hz, about 1,000 Hz, about 1,500 Hz, about 2,000 Hz, about 2,500 Hz, or about 3,000 Hz. In some embodiments, the frequency of each of the soundwaves at least about 1 Hz, about 300 Hz, about 500 Hz, about 750 Hz, about 1,000 Hz, about 1,500 Hz, about 2,000 Hz, or about 2,500 Hz. In some embodiments, the frequency of each of the soundwaves at most about 300 Hz, about 500 Hz, about 750 Hz, about 1,000 Hz, about 1,500 Hz, about 2,000 Hz, about 2,500 Hz, or about 3,000 Hz.

In some embodiments, the frequency of each of the soundwaves is about 1 KHz to about 3,000 KHz. In some embodiments, the frequency of each of the soundwaves is about 1 KHz to about 3,000 KHz. In some embodiments, the frequency of each of the soundwaves is about 1 KHz to about 250 KHz, about 1 KHz to about 750 KHz, about 1 KHz to about 1,000 KHz, about 1 KHz to about 1,250 KHz, about 1 KHz to about 1,500 KHz, about 1 KHz to about 1,750 KHz, about 1 KHz to about 2,000 KHz, about 1 KHz to about 2,250 KHz, about 1 KHz to about 2,500 KHz, about 1 KHz to about 2,750 KHz, about 1 KHz to about 3,000 KHz, about 250 KHz to about 750 KHz, about 250 KHz to about 1,000 KHz, about 250 KHz to about 1,250 KHz, about 250 KHz to about 1,500 KHz, about 250 KHz to about 1,750 KHz, about 250 KHz to about 2,000 KHz, about 250 KHz to about 2,250 KHz, about 250 KHz to about 2,500 KHz, about 250 KHz to about 2,750 KHz, about 250 KHz to about 3,000 KHz, about 750 KHz to about 1,000 KHz, about 750 KHz to about 1,250 KHz, about 750 KHz to about 1,500 KHz, about 750 KHz to about 1,750 KHz, about 750 KHz to about 2,000 KHz, about 750 KHz to about 2,250 KHz, about 750 KHz to about 2,500 KHz, about 750 KHz to about 2,750 KHz, about 750 KHz to about 3,000 KHz, about 1,000 KHz to about 1,250 KHz, about 1,000 KHz to about 1,500 KHz, about 1,000 KHz to about 1,750 KHz, about 1,000 KHz to about 2,000 KHz, about 1,000 KHz to about 2,250 KHz, about 1,000 KHz to about 2,500 KHz, about 1,000 KHz to about 2,750 KHz, about 1,000 KHz to about 3,000 KHz, about 1,250 KHz to about 1,500 KHz, about 1,250 KHz to about 1,750 KHz, about 1,250 KHz to about 2,000 KHz, about 1,250 KHz to about 2,250 KHz, about 1,250 KHz to about 2,500 KHz, about 1,250 KHz to about 2,750 KHz, about 1,250 KHz to about 3,000 KHz, about 1,500 KHz to about 1,750 KHz, about 1,500 KHz to about 2,000 KHz, about 1,500 KHz to about 2,250 KHz, about 1,500 KHz to about 2,500 KHz, about 1,500 KHz to about 2,750 KHz, about 1,500 KHz to about 3,000 KHz, about 1,750 KHz to about 2,000 KHz, about 1,750 KHz to about 2,250 KHz, about 1,750 KHz to about 2,500 KHz, about 1,750 KHz to about 2,750 KHz, about 1,750 KHz to about 3,000 KHz, about 2,000 KHz to about 2,250 KHz, about 2,000 KHz to about 2,500 KHz, about 2,000 KHz to about 2,750 KHz, about 2,000 KHz to about 3,000 KHz, about 2,250 KHz to about 2,500 KHz, about 2,250 KHz to about 2,750 KHz, about 2,250 KHz to about 3,000 KHz, about 2,500 KHz to about 2,750 KHz, about 2,500 KHz to about 3,000 KHz, or about 2,750 KHz to about 3,000 KHz. In some embodiments, the frequency of each of the soundwaves is about 1 KHz, about 250 KHz, about 750 KHz, about 1,000 KHz, about 1,250 KHz, about 1,500 KHz, about 1,750 KHz, about 2,000 KHz, about 2,250 KHz, about 2,500 KHz, about 2,750 KHz, or about 3,000 KHz. In some embodiments, the frequency of each of the soundwaves is at least about 1 KHz, about 250 KHz, about 750 KHz, about 1,000 KHz, about 1,250 KHz, about 1,500 KHz, about 1,750 KHz, about 2,000 KHz, about 2,250 KHz, about 2,500 KHz, or about 2,750 KHz. In some embodiments, the frequency of each of the soundwaves is at most about 250 KHz, about 750 KHz, about 1,000 KHz, about 1,250 KHz, about 1,500 KHz, about 1,750 KHz, about 2,000 KHz, about 2,250 KHz, about 2,500 KHz, about 2,750 KHz, or about 3,000 KHz.

At operation 630, based on the vibrational response of the blood vessel to the soundwaves at each of the plurality of different frequencies, the resonant frequency of vibration of the blood vessel may be determined (e.g., based on the frequency that produced the greatest amplitude of response). In some embodiments, the resonant frequency may be held for a moment and then recorded in memory (e.g., in computer readable medium 340).

In some embodiments, an ultrasonic sensor is used to detect the resonant frequency by capturing images of the blood vessel (e.g., the cross section) as it vibrates in response to different applied frequencies. The frequency that produces the highest amplitude of vibration may be selected (e.g., by a processing device communicatively coupled to the sensor) as the resonant frequency. In some embodiments, the ultrasonic transducer has a sampling rate of at least 3 kHz.

In some embodiments, the electroacoustic transducer and ultrasonic sensor may be placed adjacent one another and driven in synchronization. For example, a small speaker may be placed on either side of the ultrasonic sensor, and the two devices/components may be synchronously driven.

In some embodiments of operations 620 and 630, to improve the speed of finding the resonant frequency, an optimized search algorithm may be used to adjust the frequency of soundwaves output by the electroacoustic transducer in-between measurements. The search algorithm may utilize suitable boundary conditions (e.g., maximum frequency, minimum frequency, rate of frequency adjustment, etc.). The search algorithm may begin with a wide frequency scan over a large frequency range that uses large changes of frequency between measurements to find a narrower frequency range within which the resonant peak lies, and then transition to a narrow frequency scan within this narrower frequency range to find the resonant frequency. In some embodiments, the resonant frequency may be held for a moment and then recorded in memory (e.g., in computer readable medium 340).

At operation 640, the wall thickness and radius of the blood vessel (e.g., for the cross section) are determined. These parameters may be determined from the ultrasound images captured using an ultrasound sensor 320. For example, echo-mode ultrasonography may be utilized to determine the wall thickness and radius. Ultrasonic waves may be directed to the blood vessel and ultrasonic waves reflected from echogenic boundaries of the blood vessel may be received. A doppler shift of the reflected ultrasonic waves may be measured using the ultrasonic sensor, and a wave velocity of the blood vessel may be calculated based on this doppler shift. In some embodiments, multiple ultrasonic transducers may be utilized to improve imaging accuracy and/or speed. For example, one transducer could produce an ultrasonic signal while three others listen for echoes, allowing for triangulation of echoes as they are received.

At operation 650, the blood pressure is calculated based on the resonant frequency, the wall thickness, and the radius. The calculated blood pressure may establish a baseline diastolic pressure. Once an absolute diastolic pressure is established, changes in blood pressure over time (e.g., using doppler ultrasound imaging) may be measured with the ultrasound sensor(s) to extract a continuous waveform. When employing this type of differential measurement, a small but persistent inaccuracy aggregate into a large drift over the course of many heartbeats. Thus, periodic re-baselining may be required to maintain accuracy for a patient under long-term monitoring. This demonstrates another advantage of the proposed method; while other baselining techniques would require repeated application of an external pressure cuff or force probe, using the method herein, the baselining can be performed automatically at regular intervals using the electroacoustic transducer and the same ultrasonic hardware being employed to perform the continuous monitoring.

In some embodiments, device 300 or an external display system 350 may display the computed blood pressure over time. In some embodiments, a processing device 330 of continuous blood pressure measurement device 300 may perform the necessary DSP and calculations to arrive at the patient's blood pressure.

In some embodiments, the electroacoustic transducer may be omitted and the acoustic signals of varying frequencies may be generated by pulsing the ultrasonic sensor at varying frequencies to deliver the energy to the blood vessel. This alternative design may have the advantage of using a single sensor to produce both the resonant response and capture ultrasound imaging data.

In some embodiments, the system comprises an ultrasound platform for driving one or more ultrasonic transducers. In some embodiments, the ultrasound platform processes and/or conditions signals received by one or more ultrasound transducers.

## II. ADHESIVE PATCH MONITORING DEVICE

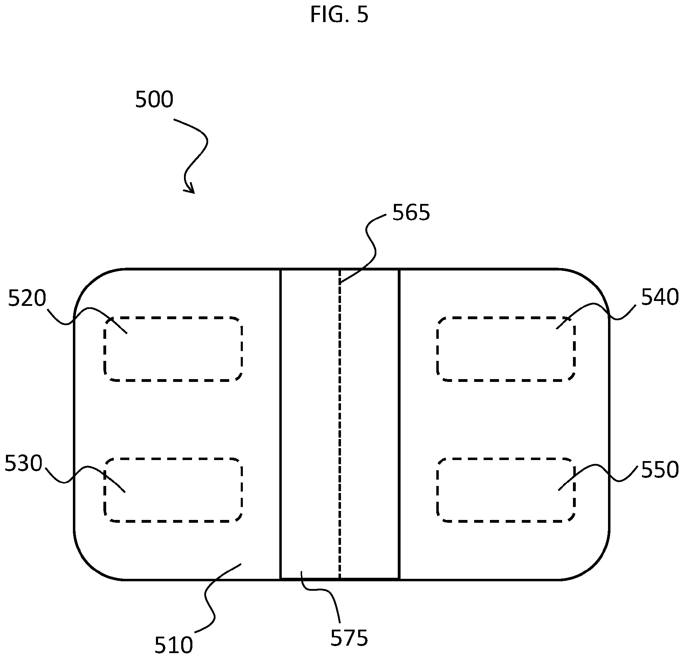

In some embodiments, depicted by FIG. 5, a continuous blood pressure measurement device 500 may be incorporated into a substrate 510. As illustrated, the device 500 may include one or more alignment lines 565 or other markings for aligning one or more sensors or transducers 520, 530, 540, 550 of the device 500 with an artery of a patient prior to taking actuating transducer 310 and taking ultrasound measurements. In some embodiments, a transparent window 575 is provided for aligning one or more sensors or transducers 520, 530, 540, 550 of the device 500 with an artery of a patient prior to taking actuating transducer 310 and taking ultrasound measurements. In some embodiments, the device may comprise a transparent window 575 for viewing the artery behind the substrate during placement. A combination of a transparent window and alignment marker may further facilitate placement of the device.

Additionally, substrate 510 may include an adhesive that used to hold substrate in place and in alignment with the patient's artery. During application, the adhesive may be exposed by peeling away a paper backing. Adhesives may include a rubber, acrylic, or acrylic blend adhesive. In some embodiments, the device can be placed on the patient's neck to measure blood pressure through the carotid artery. Similar measurements may be made on the radial or ulnar arteries. By virtue of some embodiments, the measurement of blood pressure may be noninvasive, reliable, fast, and provide continuous measurement, thus considering beat to beat variation. These continuous variations may be presented to a user on a display system 350 as described above.

In some embodiments, the sensors and transducers of the device 500 include at least one ultrasound transducer and at least one electroacoustic transducer. Inclusion of more than one ultrasound transducers may allow for increased resolution as described herein. In some embodiments, a device comprises two ultrasound transducers and one electroacoustic transducer. In some embodiments the patch comprises three ultrasound transducers and one electroacoustic transducer. In some embodiments the patch comprises four ultrasound transducers. Each transducer 520, 530, 540, 550 may be configured as a transmitter, receiver, or both a transmitter and receiver (transceiver). In some embodiments, transducer 520 is configured as a transmitter and transducer 540 is configured as a receiver. In some embodiments, transducer 520 is configured as a transmitter and transducer 530 is configured as a receiver. In some embodiments, transducer 520 is configured as a transmitter and transducer 550 is configured as a receiver. In some embodiments, transducer 530 is configured as a transmitter and transducer 520 is configured as a receiver. In some embodiments, transducer 530 is configured as a transmitter and transducer 540 is configured as a receiver. In some embodiments, transducer 530 is configured as a transmitter and transducer 550 is configured as a receiver. In some embodiments, transducers 520 and 530 are configured as transmitters and transducers 540 and 550 are configured as receivers. In some embodiments, transducers 530 and 540 are configured as transmitters and transducers 520 and 550 are configured as receivers. In some embodiments, transducers 520 and 550 are configured as transmitters and transducers 530 and 540 are configured as receivers. In some embodiments, transducers 520 and 540 are configured as transmitters and transducers 530 and 550 are configured as receivers. In some embodiments, transducer 520 is configured as transmitters and transducers 530 and 540 are configured as receivers. In some embodiments, transducer 530 is configured as transmitters and transducers 550 and 540 are configured as receivers.

In some embodiments, the one or more transducers is incorporate into a wearable device. Wearable devices may include a wristband or watch, wherein the one or more transducers are directed to a brachial artery to determine a mean arterial pressure. In some embodiments, the substrate comprising the one or more transducers is incorporated into a wearable device. The wearable device may communicate wirelessly with an external computing device to display or record data.

In some embodiments, the device comprises four ultrasound transducers and an electroacoustic transducer or speaker. In some embodiments, images or signals captured by the ultrasound transducers are input into a rotational matrix. Rotation and/or skew matrices may be applied to properly orient the received signals for convolution, deconvolution, or normalization. Normalization may be applied to received signals, as disclosed herein. In some embodiments, a skew parameter is applied to the rotational matrices.

Each ultrasonic transducer of the plurality of ultrasonic transducers may be configured to transmit a transmitted ultrasonic imaging signal to an object. Each ultrasonic transducer of the plurality may be configured to transmit a transmitted ultrasonic imaging signal having a frequency of about 100 kHz, about 200 kHz, about 300 kHz, about 400 kHz, about 500 kHz, about 650 kHz, about 700 kHz, about 800 kHz, about 850 kHz, about 900 kHz, about 1 MHz, about 2 MHz, about 3 MHz, about 5.5 MHz, about 6 MHz, about 8 MHz, about 11 MHz, about 15 MHz, about 20 MHz, about 25 MHz, or about 30 MHz Each ultrasonic transducer of the plurality may be configured to transmit a transmitted ultrasonic imaging signal having a frequency that is within a range defined by any two of the preceding values.

Each ultrasonic transducer of the plurality of ultrasonic transducers may be configured to receive a received ultrasonic imaging signal from an object. Each ultrasonic transducer of the plurality may be configured to receive a received ultrasonic imaging signal having a frequency of about 100 kHz, about 200 kHz, about 300 kHz, about 400 kHz, about 500 kHz, about 650 kHz, about 700 kHz, about 800 kHz, about 850 kHz, about 900 kHz, about 1 MHz, about 2 MHz, about 3 MHz, about 5.5 MHz, about 6 MHz, about 8 MHz, about 11 MHz, about 15 MHz, about 20 MHz, about 25 MHz, or about 30 MHz Each ultrasonic transducer of the plurality may be configured to receive a received ultrasonic imaging signal having a frequency that is within a range defined by any two of the preceding values.

Each ultrasonic transducer of the plurality may be configured both to transmit and to receive. Each ultrasonic transducer of the plurality may be configured to transmit transmitted ultrasonic imaging signals or receive received ultrasonic imaging signals at a frequency that is the same as one or more of the frequencies transmitted or received by other ultrasonic transducer of the plurality. Each ultrasonic transducer of the plurality may be configured to transmit transmitted ultrasonic imaging signals or receive received ultrasonic imaging signals at a frequency that is different from all of frequencies transmitted or received by all other ultrasonic transducer of the plurality. Each of the ultrasonic transducer of the plurality may be configured to transmit or receive at the same time as one or more other ultrasonic transducer of the plurality.

For instance, a first transmitted imaging signal of a first ultrasonic transducer of the plurality may have a frequency of about 100 kHz, about 200 kHz, about 300 kHz, about 400 kHz, about 500 kHz, about 650 kHz, about 700 kHz, about 800 kHz, about 850 kHz, about 900 kHz, about 1 MHz, about 2 MHz, about 3 MHz, about 5.5 MHz, about 6 MHz, about 8 MHz, or about 11 MHz A second transmitted imaging signal of a second ultrasonic transducer of the plurality may have a frequency that is in a range from about 0.5 MHz to about 30 MHz. A first received imaging signal of a first ultrasonic transducer of the plurality may have a frequency of about 100 kHz, about 200 kHz, about 300 kHz, about 400 kHz, about 500 kHz, about 650 kHz, about 700 kHz, about 800 kHz, about 850 kHz, about 900 kHz, about 1 MHz, about 2 MHz, about 3 MHz, about 5.5 MHz, about 6 MHz, about 8 MHz, or about 11 MHz A second received imaging signal of a second ultrasonic transducer of the plurality may have a frequency that is in a range from about 0.5 MHz to about 30 MHz.

In another example, a first transmitted imaging signal of a first ultrasonic transducer of the plurality may have a frequency that is in a range from about 0.5 MHz to about 30 MHz A second transmitted imaging signal of a second ultrasonic transducer of the plurality may have a frequency that is in a range from about 0.5 MHz to about 30 MHz, but that is different from the frequency of the first transmitted imaging signal. A first received imaging signal of a first ultrasonic transducer of the plurality may have a frequency that is in a range from about 0.5 MHz to about 30 MHz A second received imaging signal of a second ultrasonic transducer of the plurality may have a frequency that is in a range from about 0.5 MHz to about 30 MHz, but that is different from the frequency of the first received imaging signal.

A third, fourth, fifth, sixth, seventh, eighth, ninth, tenth, eleventh, twelfth, thirteenth, fourteenth, fifteenth, or sixteenth transmitted imaging signal of a third, fourth, fifth, sixth, seventh, eighth, ninth, tenth, eleventh, twelfth, thirteenth, fourteenth, fifteenth, or sixteenth ultrasonic transducer, respectively, may have a frequency that is about 100 kHz, about 200 kHz, about 300 kHz, about 400 kHz, about 500 kHz, about 650 kHz, about 700 kHz, about 800 kHz, about 850 kHz, about 900 kHz, about 1 MHz, about 2 MHz, about 3 MHz, about 5.5 MHz, about 6 MHz, about 8 MHz, or about 11 MHz. The third, fourth, fifth, sixth, seventh, eighth, ninth, tenth, eleventh, twelfth, thirteenth, fourteenth, fifteenth, or sixteenth transmitted imaging signal may have a frequency that is within a range described by any two of the preceding values. The third, fourth, fifth, sixth, seventh, eighth, ninth, tenth, eleventh, twelfth, thirteenth, fourteenth, fifteenth, or sixteenth transmitted imaging signal may have a value that is in a range from about 0.5 MHz to about 30 MHz. The third, fourth, fifth, sixth, seventh, eighth, ninth, tenth, eleventh, twelfth, thirteenth, fourteenth, fifteenth, or sixteenth transmitted imaging signal may have a frequency that is different from one or more of the frequencies of the first and second transmitted imaging signals.

A third, fourth, fifth, sixth, seventh, eighth, ninth, tenth, eleventh, twelfth, thirteenth, fourteenth, fifteenth, or sixteenth received imaging signal of a third, fourth, fifth, sixth, seventh, eighth, ninth, tenth, eleventh, twelfth, thirteenth, fourteenth, fifteenth, or sixteenth ultrasonic transducer, respectively, may have a frequency that is about 100 kHz, about 200 kHz, about 300 kHz, about 400 kHz, about 500 kHz, about 650 kHz, about 700 kHz, about 800 kHz, about 850 kHz, about 900 kHz, about 1 MHz, about 2 MHz, about 3 MHz, about 5.5 MHz, about 6 MHz, about 8 MHz, or about 11 MHz. The third, fourth, fifth, sixth, seventh, eighth, ninth, tenth, eleventh, twelfth, thirteenth, fourteenth, fifteenth, or sixteenth received imaging signal may have a frequency that is within a range described by any two of the preceding values. The third, fourth, fifth, sixth, seventh, eighth, ninth, tenth, eleventh, twelfth, thirteenth, fourteenth, fifteenth, or sixteenth received imaging signal may have a value that is in a range from about 0.5 MHz to about 30 MHz. The third, fourth, fifth, sixth, seventh, eighth, ninth, tenth, eleventh, twelfth, thirteenth, fourteenth, fifteenth, or sixteenth received imaging signal may have a frequency that is different from one or more of the frequencies of the first and second transmitted imaging signals.

Each ultrasonic transducer of the plurality of transducers may transmit transmitted ultrasonic imaging signals or receive received ultrasonic imaging signals within a bandwidth. The first ultrasonic transducer may have a first bandwidth and the second ultrasonic transducer may have a second bandwidth. The first bandwidth and the second bandwidth may overlap. The first bandwidth and the second bandwidth may partially overlap. The first bandwidth and the second bandwidth may not overlap. Similarly, the third, fourth, fifth, sixth, seventh, eighth, ninth, tenth, eleventh, twelfth, thirteenth, fourteenth, fifteenth, or sixteenth ultrasonic transducers may have third, fourth, fifth, sixth, seventh, eighth, ninth, tenth, eleventh, twelfth, thirteenth, fourteenth, fifteenth, or sixteenth bandwidths, respectively. Any one of the first, second, third, fourth, fifth, sixth, seventh, eighth, ninth, tenth, eleventh, twelfth, thirteenth, fourteenth, fifteenth, or sixteenth bandwidths may overlap one another. Any one of the first, second, third, fourth, fifth, sixth, seventh, eighth, ninth, tenth, eleventh, twelfth, thirteenth, fourteenth, fifteenth, or sixteenth bandwidths may partially overlap one another. Any one of the first, second, third, fourth, fifth, sixth, seventh, eighth, ninth, tenth, eleventh, twelfth, thirteenth, fourteenth, fifteenth, or sixteenth bandwidths may not overlap one another.

The received imaging signals may be subjected to pre-processing operations. For instance, a first received imaging signal may form a basis for normalizing other received imaging signals. A second received imaging signal may be normalized by the first received imaging signal. A third, fourth, fifth, sixth, seventh, eighth, ninth, tenth, eleventh, twelfth, thirteenth, fourteenth, fifteenth, or sixteenth received imaging signal may be normalized by the first received imaging signal.

The system may comprise a transmit (Tx) generator. The transmit generator may comprise a host computer, software-defined radio, and ultrasound signal processing circuit (USPC), as disclosed herein. The Tx generator may be a Tx beamformer. The Tx generator may be configured to operate any one of the ultrasonic transducers to transmit a first, second, third, or fourth transmitted ultrasonic imaging signal, respectively. The Tx generator may operate any two or more of the first, second, third, or fourth ultrasonic imaging transducers simultaneously. The system may further comprise an image synthesis module. The image synthesis module may comprise a receive (Rx) beamformer. The Rx beamformer may be configured to operate any one of ultrasonic transducers to receive a first, second, third, or fourth received ultrasonic imaging signal, respectively. The image synthesis module may subject the received ultrasonic imaging signals to an ultrasonic image reconstruction operation. For instance, the image synthesis module may subject the received ultrasonic imaging signals to a delay and sum operation. The image synthesis module may subject the received ultrasonic imaging signals to any ultrasonic image reconstruction operation. The Tx generator may be configured to operate fifth, sixth, seventh, eighth, ninth, tenth, eleventh, twelfth, thirteenth, fourteenth, fifteenth, or sixteenth ultrasonic transducers to transmit fifth, sixth, seventh, eighth, ninth, tenth, eleventh, twelfth, thirteenth, fourteenth, fifteenth, or sixteenth transmitted ultrasonic imaging signals, respectively. The Tx generator may operate any two or more of the first, second, third, fourth, fifth, sixth, seventh, eighth, ninth, tenth, eleventh, twelfth, thirteenth, fourteenth, fifteenth, or sixteenth ultrasonic imaging transducers simultaneously. Similar, the Rx beamformer may be configured to operate fifth, sixth, seventh, eighth, ninth, tenth, eleventh, twelfth, thirteenth, fourteenth, fifteenth, or sixteenth ultrasonic transducers to transmit fifth, sixth, seventh, eighth, ninth, tenth, eleventh, twelfth, thirteenth, fourteenth, fifteenth, or sixteenth transmitted ultrasonic imaging signals, respectively.

The Tx generator may be configured to operate any one of the first, second, third, fourth, fifth, sixth, seventh, eighth, ninth, tenth, eleventh, twelfth, thirteenth, fourteenth, fifteenth, or sixteenth ultrasonic transducers to transmit in sequence.

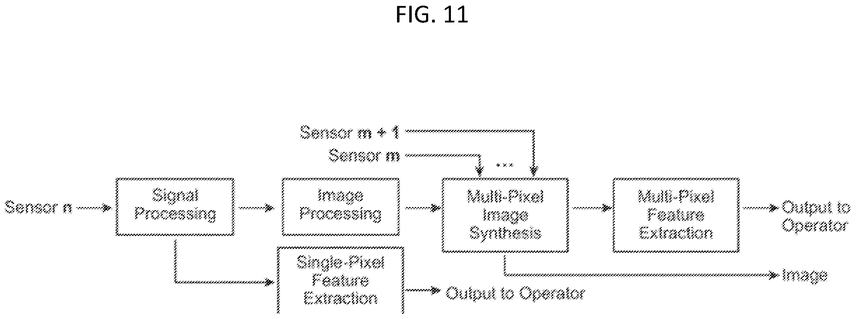

FIG. 11 schematically illustrates a method of forming ultrasonic images from a plurality of ultrasonic transducers. The method may utilize measurements from a plurality of ultrasonic imaging sensors. The method may utilize single-pixel and multi-pixel image processing techniques. In the single-pixel case, an n-th ultrasonic imaging measurement (where n is a positive integer) may be input to a signal processing unit. The signal processing unit may apply any ultrasonic signal processing procedure to the n-th ultrasonic imaging measurement. The signal processing unit may output a signal processed measurement to an image processing unit and to a single-pixel feature extraction unit. The image processing unit may apply any ultrasonic image processing procedure. The single-pixel feature extraction unit may apply any ultrasonic single-pixel feature extraction procedure. The single-pixel feature extraction unit may output an extracted feature to an operator.

In the multi-pixel case, an m-th and an (m+1)-th (where m and m+1 are positive integers) ultrasonic imaging measurement may be input to a multi-pixel image synthesis unit and to a multi-pixel feature extraction unit. The image synthesis unit may apply any ultrasonic image synthesis procedure. The multi-pixel feature extraction unit may apply any ultrasonic multi-pixel feature extraction procedure. The multi-feature extraction unit may output an extracted feature to an operator. Multi-pixel extraction may be utilized to build 3D pixels (i.e. voxels).

In the multi-pixel case, image processing methods such as 2-dimensional smoothing filters, Harr filters, Gaussian filters, and integrators may be used to improve the recorded image. Furthermore, each pixel may be filtered in the time-domain to accentuate signal features. A single or multiple Butterworth, Chebyshev, and elliptic filters can be used to suppress noise and enhance feature extraction.

Sensors of the device may further comprise a temperature sensor, an optical sensor, an electrical sensor, a chemical sensor and an electrochemical sensor. In some embodiments, additional sensors allow measurement of body temperature, a respiration rate, a blood pressure level, and a blood oxygenation saturation (spO$_2$) level.

The stethoscope device may comprise a first ultrasonic transducer, a light source, and a light detector. The stethoscope device may optionally comprise a second ultrasonic transducer. The first ultrasonic transducer may operate in a Tx mode. The second ultrasonic transducer may operate in a receive mode. The stethoscope device may be placed above the skin of a subject (such as skin in a subject's arm). As a bolus of blood travels through an artery beneath the skin of the subject, the stethoscope device may transmit an ultrasonic signal from the first ultrasonic transducer and an optical signal from the light source. The ultrasonic signal or the optical signal may be scattered, dispersed, or reflected from the bolus. The scattered, dispersed, or reflected ultrasonic signal or optical signal may be detector by the second ultrasonic transducer or the light detector, respectively. The intensity of the scattered, dispersed, or reflected ultrasonic signal or optical signal may be compared to the intensity of the transmitted ultrasonic signal or the transmitted optical signal, respectively. These measurements may yield a velocity of the blood bolus as measured by the ultrasonic imaging signal and the optical signal, respectively. The velocity of the blood bolus as measured by the ultrasonic imaging signal may be normalized by the velocity of the blood bolus as measured by the optical signal, or vice versa. These values may be synthesized and correlated to determine one or more physiological parameters of the subject, such as the subject's heart rate, blood pressure, or respiration.

## III. METHODS FOR INCREASING ULTRASOUND RESOLUTION

Ultrasonic tissue imaging may be plagued by poor optical and acoustic transmissivity as well as beam scattering. To address such difficulties and increase resolution, a method of multi-wavelength ultrasound imaging by collection of information from a plurality of ultrasound sensors, where each sensor has an ultrasound transducer with a unique resonance frequency may be utilized. By virtue of resonating physiology with multiple ultrasound sensors operating at distinct resonance frequencies, ultrasound emitted by the plurality of transducers may simultaneously penetrate various depths in a given cross-sectional area of physiology (e.g., tissue). In particular, ultrasound sensors having acoustic frequency responses may be actuated in a time interleaved fashion such that each sensor may be used to image a physiological surface at a unique depth. This may enable high resolution tomography without needing to increase the gain or the number of ultrasound imaging sensors. Furthermore, each sensor may be simultaneously actuated with a combination of other sensors to generate higher order harmonics which allow for sub-pixel feature resolution (i.e., super resolution imaging).

As used herein to refer to a transducer, the term "resonant frequency" generally refers to an operating frequency at which the transducer most efficiently converts electrical energy into mechanical energy. For example, in the context of a piezoelectric transducer, the term resonant frequency may refer to an operating frequency at which the piezoelectrical material most readily vibrates and converts electrical energy into mechanical energy most efficiently.

Ultrasound that enters tissue may be transmitted, attenuated or reflected. While higher frequency ultrasound may provide for a higher resolution signal, it may provide poor depth penetration of imaged tissue. Conversely, while lower frequency ultrasound may provide for a lower resolution signal, it may provide better depth penetration of imaged tissue. To overcome these limitations of conventional ultrasound imaging systems, each ultrasound sensor may have a transducer configured with a unique resonance frequency. Specifically, the ultrasound transducers may be selected such that their acoustic frequency responses are non-overlapping. By actuating the transducers in a time interleaved fashion, each sensor may be used to image a surface of a sample at a unique depth. Furthermore, each transducer may be simultaneously actuated with a combination of other transducers to generate higher order harmonics that allow for sub-pixel feature resolution.

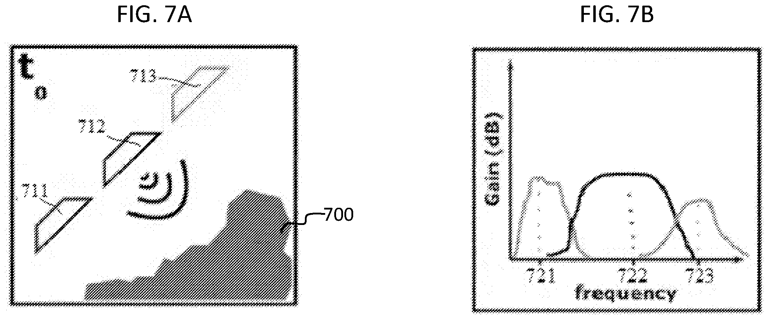

As shown by FIG. 7A, selecting transducers 711, 712, 713 having frequencies which are partially overlapping may allow for self-normalization and cross-correlation of signals. In some embodiments, by selecting sensor transducers that are adjacent and partially overlapping in frequency response, the measurement of any pair of sensors may be normalized, thereby reducing systemic sources of noise and significantly increasing signal integrity. Additionally, the simultaneous interrogation of a surface with transducers with distinct resonance frequencies can be used to generate super-resolution ultrasound images of an object 700. In some embodiments, the frequency overlap between sensors 711, 712, 713 may be configured to be about 200 KHz or less. In some embodiments, the frequency overlap between sensors may be configured such that a frequency response range of one sensor does not overlap with a resonant frequency of another sensor. For example, as depicted by FIG. 7B, a first sensor 711 having a resonant frequency 721 and a second sensor 712 having a resonant frequency 722 partially overlap in frequency response. Additionally, a second sensor 712 and a third sensor 713 having a resonant frequency 723 partially overlap in frequency response.

In some embodiments, the frequency overlap between sensors is about 10 KHz to about 500 KHz. In some embodiments, the frequency overlap between sensors is about 10 KHz to about 20 KHz, about 10 KHz to about 30 KHz, about 10 KHz to about 40 KHz, about 10 KHz to about 50 KHz, about 10 KHz to about 75 KHz, about 10 KHz to about 100 KHz, about 10 KHz to about 150 KHz, about 10 KHz to about 200 KHz, about 10 KHz to about 300 KHz, about 10 KHz to about 400 KHz, about 10 KHz to about 500 KHz, about 20 KHz to about 30 KHz, about 20 KHz to about 40 KHz, about 20 KHz to about 50 KHz, about 20 KHz to about 75 KHz, about 20 KHz to about 100 KHz, about 20 KHz to about 150 KHz, about 20 KHz to about 200 KHz, about 20 KHz to about 300 KHz, about 20 KHz to about 400 KHz, about 20 KHz to about 500 KHz, about 30 KHz to about 40 KHz, about 30 KHz to about 50 KHz, about 30 KHz to about 75 KHz, about 30 KHz to about 100 KHz, about 30 KHz to about 150 KHz, about 30 KHz to about 200 KHz, about 30 KHz to about 300 KHz, about 30 KHz to about 400 KHz, about 30 KHz to about 500 KHz, about 40 KHz to about 50 KHz, about 40 KHz to about 75 KHz, about 40 KHz to about 100 KHz, about 40 KHz to about 150 KHz, about 40 KHz to about 200 KHz, about 40 KHz to about 300 KHz, about 40 KHz to about 400 KHz, about 40 KHz to about 500 KHz, about 50 KHz to about 75 KHz, about 50 KHz to about 100 KHz, about 50 KHz to about 150 KHz, about 50 KHz to about 200 KHz, about 50 KHz to about 300 KHz, about 50 KHz to about 400 KHz, about 50 KHz to about 500 KHz, about 75 KHz to about 100 KHz, about 75 KHz to about 150 KHz, about 75 KHz to about 200 KHz, about 75 KHz to about 300 KHz, about 75 KHz to about 400 KHz, about 75 KHz to about 500 KHz, about 100 KHz to about 150 KHz, about 100 KHz to about 200 KHz, about 100 KHz to about 300 KHz, about 100 KHz to about 400 KHz, about 100 KHz to about 500 KHz, about 150 KHz to about 200 KHz, about 150 KHz to about 300 KHz, about 150 KHz to about 400 KHz, about 150 KHz to about 500 KHz, about 200 KHz to about 300 KHz, about 200 KHz to about 400 KHz, about 200 KHz to about 500 KHz, about 300 KHz to about 400 KHz, about 300 KHz to about 500 KHz, or about 400 KHz to about 500 KHz. In some embodiments, the frequency overlap between sensors is about 10 KHz, about 20 KHz, about 30 KHz, about 40 KHz, about 50 KHz, about 75 KHz, about 100 KHz, about 150 KHz, about 200 KHz, about 300 KHz, about 400 KHz, or about 500 KHz. In some embodiments, the frequency overlap between sensors is at least about 10 KHz, about 20 KHz, about 30 KHz, about 40 KHz, about 50 KHz, about 75 KHz, about 100 KHz, about 150 KHz, about 200 KHz, about 300 KHz, or about 400 KHz. In some embodiments, the frequency overlap between sensors is at most about 20 KHz, about 30 KHz, about 40 KHz, about 50 KHz, about 75 KHz, about 100 KHz, about 150 KHz, about 200 KHz, about 300 KHz, about 400 KHz, or about 500 KHz.

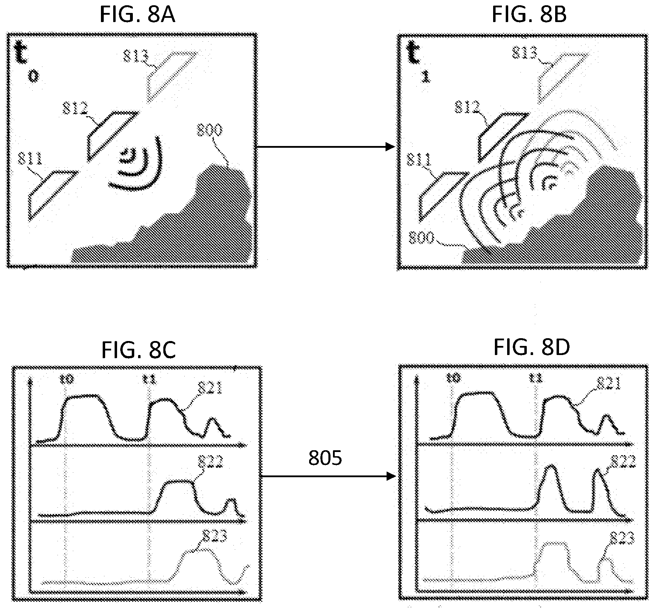

FIGS. 8A-8D depicts a technique for cross-correlation in an ultrasound measurement device. As depicted, the cross-correlation of signals from any pair of sensors 811, 812, 813 whose frequency responses overlap allows for a redundant measurement of a voxel. Specifically, the transducers of the sensors may be actuated in a time-interleaved fashion. For each actuation, for example an actuation of sensor 812 to transmit a signal to an object 800, the received echo may be measured by all three transducers 811, 812, 812 as depicted in FIG. 8B to obtain received signals 821, 822, 823 on all three transducers as depicted in FIG. 8C. By looking at the correlation of the received signals on all three sensors, the signals may be normalized. FIG. 8C illustrates the event where the echo from actuation of a second sensor 812 is measured by all three sensors. In some embodiments, the frequency response of a first sensor 811 and a second sensor 812 is convolved in the frequency domain with the received echo waveform. When deconvolved, at step 805 the measurement from the third sensor may be correlated with the measurement from the second sensor while using the measurement of first sensor to normalize the measurement of the second sensor. As depicted by FIG. 8D, a much sharper peak in a signal monitored by the second sensor is obtained, and those sharper peaks may be achieved by the aforementioned cross-frequency normalization.

## IV. SIGNAL GENERATION, TRANSMISSION, AND RECEIVING

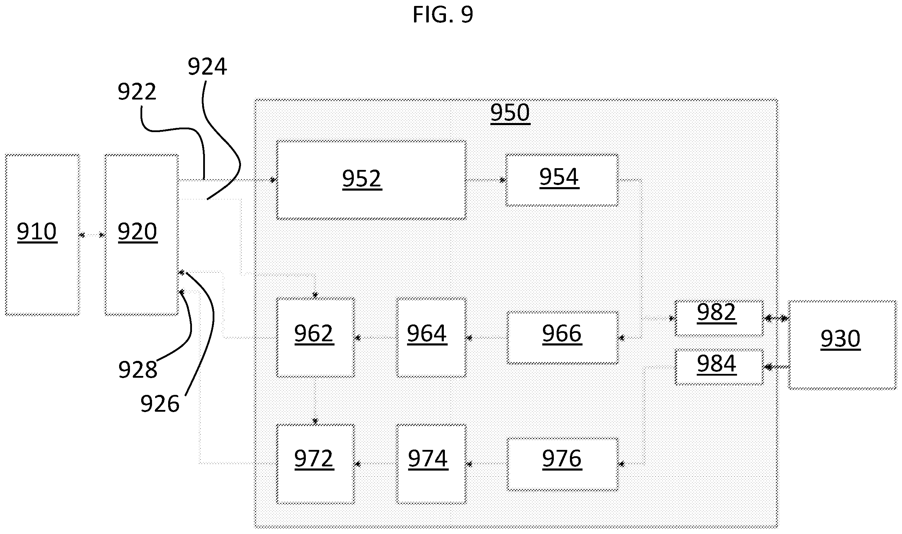

In some embodiments, a circuit is utilized to process ultrasound signals. FIG. 9 depicts an ultrasound signal processing circuit (USPC) 950 configured to process and/or condition ultrasound signals transmitted between one or more ultrasound transducers 930 and a software-defined radio system 920, according to some embodiments. The system used to generate and receive ultrasound signals, as depicted in FIG. 9, may be correspond to a transmit (Tx) generator and/or a receive (Rx) beamformer as described herein. One or more signals transmitted by the software-defined radio system may be referred to as the outputs of the software-defined radio system. One or more signals transmitted by the software-defined radio system may be referred to as the inputs of the software-defined radio system.

In some embodiments, the software-defined radio system 920 transmits one or more signals 922, 924 to the ultrasound signal processing circuit (USPC) 950. In some embodiments, a first transmission signal 922 comprises a transmission pulse or reference clock. In some embodiments, a second transmission 924 signal comprises a gain ramp. In some embodiments, the transmission pulse 922 is a low-voltage transmission pulse. In some embodiments, the transmission pulse 922 is a +/- 4 volt signal.

In some embodiments, a first transmission signal 922 is received by a high-voltage amplifier 952. In some embodiments, a transmission pulse or reference clock is input into the high-voltage amplifier 952. In some embodiments, an amplified signal from the high-voltage amplifier 952 passes through a diode 954. In some embodiments, the first transmission signal 922 is then transmitted to a first multiplexer 982. In some embodiments, the first transmission signal 922 is a transmission pulse or reference clock transmitted to the first multiplexer 982. In some embodiments, diode 954 is an anti-parallel diode configured to block received signals from the ultrasound transducer 930 or multiplexer 982 to the high-voltage amplifier 952. A high-voltage amplifier may be a discrete amplifier. In some embodiments, the high voltage amplifier output signal is a +/- 100 volt amplifier. In some embodiments, the high voltage amplifier 952 creates a +/- 100 volt device signal from a low voltage output of the software-defined radio 920.

In some embodiments, an output from the first multiplexer 928 is transmitted to the one or more ultrasound transducers 930. In some embodiments, the output from the first multiplexer 928 is received by a first pixel group of the ultrasound transducers 930. The ultrasound transducers 930 may then transmit a first pixel group ultrasonic signal to an object. The object may then reflect the transmitted ultrasonic signal and the ultrasound transducers 930 may receive the reflected signal. In some embodiments the reflected signal is received as part of a first pixel group. In some embodiments, the reflected signal is received as part of a second pixel group. In some embodiments, the reflected signal forms both a first pixel group and a second pixel group. The ultrasound transducers 930 may then transmit the received reflected signal as an electrical signal. The received reflected signal from a first pixel group may be considered a first received signal. In some embodiments, the first received signal may be received from the transducers 930 by the first multiplexer 982. The received reflected signal from a second pixel group may be considered a second received signal. In some embodiments, the second received signal may be received from the transducers 930 by the second multiplexer 984. In some embodiments, signals are wirelessly transmitted between the multiplexers 982, 984 and the one or more ultrasound transducers 930. A wireless communication link between the multiplexers 982,984 and ultrasound transducers 930 may be a wireless mode such as a Bluetooth® or Bluetooth® low energy (LE) link, a Wi-Fi® link, a ZigBee link, or some other suitable wireless communication link. In some embodiments, a wired communication link between the multiplexers 982,984 and ultrasound transducers 930 may be achieved using a wired transmitter or other suitable wired interface. For example, data may be transferred using a USB-C connector, a USB 2.x or 3.x connector, a micro-USB connector, a THUNDERBOLT connector, an Ethernet cable, etc.

One or more signals transmitted to the ultrasound transducers may be referred to as the inputs of the ultrasound transducers. One or more signals transmitted by the ultrasound transducers may be referred to as the outputs of ultrasound transducers. The outputs of the ultrasound transducers may correspond to the reflected ultrasound signals measured or detected by the ultrasound transducers.

In some embodiments, the first multiplexer 982 transmits the first received signal from the first pixel group to a first low-noise amplifier 964. In some embodiments, the first received signal passes through a high-voltage blocker 966. The high voltage blocker 966 may prevent signals from the high-voltage amplifier 952 from passing to the first low-noise amplifier. The blocker 966 may be a high voltage protection transmit/ receive switch (T/R switch), such as a MD0100. In some embodiments, the first low-noise amplifier 964 is a low noise, single-ended, linear in dB, general purpose variable gain amplifier, such as an AD8336. In some embodiments, the first received signal is then received by a first variable gain amp 962. In some embodiments, variable gain amp 962 is a low noise, single-ended, linear in dB, general purpose variable gain amplifier, such as an AD8336. In some embodiments, the first low-noise amplifier 964 and the first variable gain amplifier 962 utilize the same chip type.

In some embodiments, the second multiplexer 984 transmits the first received signal from the second pixel group to a second low-noise amplifier 974. In some embodiments, diode 976 is prevents a transmitted signal from being output to the second multiplexer 984. In some embodiments, diode 976 is an anti-parallel diode. In some embodiments, the second low-noise amplifier 974 is a low noise, single-ended, linear in dB, general purpose variable gain amplifier, such as an AD8336. In some embodiments, the second received signal is then received by a second variable gain amp 972. In some embodiments, variable gain amp 972 is a low noise, single-ended, linear in dB, general purpose variable gain amplifier, such as an AD8336. In some embodiments, the first low-noise amplifier 964, the first variable gain amplifier 962, the second low-noise amplifier 974, and the second variable gain amplifier 972 utilize the same chip type. In some embodiments, amplifiers 962, 964, 972, 974 receive faint return pulses an amplify them according to a gain ramp signal. In some embodiments, 962, 964, 972, 974 receive faint return pulses an amplify them according to a second transmission signal 924 which is gain ramp signal. In some embodiments the amplification of the ramp signal corresponds to the time since the input pulse. In some embodiments, the rate or ration of amplification is determined by the time since the initial pulse of the gain ramp signal was emitted. In some embodiments, the return pulses are the received signals.

In some embodiments, a second transmission 924 signal is transmitted to a first variable gain amplifier 962. In some embodiments, the signal output by the first variable gain amplifier 962 is received by the first receive channel 926 of the software-defined radio system 920 and the second variable gain amplifier 972. In some embodiments, a signal output by the second variable gain amplifier 972 is received by the second receive channel 928 of the software-defined radio system 920.

In some embodiments, the first multiplexer 982 is a 16-channel multiplexer. In some embodiments, the second multiplexer 984 is a 16-channel multiplexer. In some embodiments, the first pixel group comprises a 16 pixels. In some embodiments, the second pixel group comprises a 16 pixels. In some embodiments, the first pixel group functions as a transmit and receive (transceiver) group. In some embodiments, the second pixel group functions as a receive only group. In some embodiments, the resolution of the system is 14 bits. In some embodiments, the resolution of the system is 16 bits. In some embodiments, the system is module and additional hardware may be utilized to increase the channel number. In some embodiments, the system may comprise 16 channels to 2,048 channels. In some embodiments, the system may comprise 16 channels to 32 channels, 16 channels to 64 channels, 16 channels to 128 channels, 16 channels to 256 channels, 16 channels to 512 channels, 16 channels to 1,024 channels, 16 channels to 2,048 channels, 32 channels to 64 channels, 32 channels to 128 channels, 32 channels to 256 channels, 32 channels to 512 channels, 32 channels to 1,024 channels, 32 channels to 2,048 channels, 64 channels to 128 channels, 64 channels to 256 channels, 64 channels to 512 channels, 64 channels to 1,024 channels, 64 channels to 2,048 channels, 128 channels to 256 channels, 128 channels to 512 channels, 128 channels to 1,024 channels, 128 channels to 2,048 channels, 256 channels to 512 channels, 256 channels to 1,024 channels, 256 channels to 2,048 channels, 512 channels to 1,024 channels, 512 channels to 2,048 channels, or 1,024 channels to 2,048 channels. In some embodiments, the system may comprise 16 channels, 32 channels, 64 channels, 128 channels, 256 channels, 512 channels, 1,024 channels, or 2,048 channels. In some embodiments, the system may comprise at least 16 channels, 32 channels, 64 channels, 128 channels, 256 channels, 512 channels, or 1,024 channels. In some embodiments, the system may comprise at most 32 channels, 64 channels, 128 channels, 256 channels, 512 channels, 1,024 channels, or 2,048 channels.

In some embodiments, the software-defined radio system 920 is a Universal Software Radio Peripheral (USRP), such as a National Instruments USRP N210. In some embodiments, the software-defined radio system 920 connects to a host computer 910. In some embodiments, the radio system 920 connects to the computer 910 through a high-speed link. In some embodiments, the high-speed link comprises an ethernet connection. In some embodiments, the ethernet connection is a gigabit ethernet connection which may allow for sampling rates up 50 MS/s (Mega Samples per second). In some embodiments, a host-based software is loaded onto the computer 910 and utilized to control the radio system hardware and transmit/receive data. In some embodiments, the general functionality of a host computer is implemented into the radio system 920 with an embedded processor that allows the radio system 920 to operate in a stand-alone fashion.

In some embodiments, connection to a computer 910 comprises a universal serial bus (USB) connection, a fiber optical connection, a peripheral component interconnect expresses (PCle) connection, or other suitable connection. In some embodiments, connection to a computer 910 may allow the system to utilize the rapid access memory (RAM) of the computer. In some embodiments, an embedded processor may comprise a RAM buffer. In some embodiments, the RAM buffer may comprise 4, 8, 16, 32, 64, 80, 128, or 256 gigabytes (GB).

In some embodiments, the software-defined radio system 920 comprises a motherboard. The motherboard may provide the following subsystems: clock generation and synchronization, a field-programmed gate array (FPGA), one or more analog-to-digital converters (ADCs), one or more digital-to-analog converters (DACs), a host processor interface, and a power regulation. In some embodiments, a modular front-end, or daughterboard, is used for analog operations such as up/down-conversion, filtering, and other signal conditioning. This modularity may permit the software-defined radio system 920 to serve applications that operate between DC and 6 GHz. In some embodiments, the system operates between DC and 5MHz. In some embodiments, the system operates only down to 1 MHz, which may be considered the limit of a normal ultrasonic signal. In some embodiments, the system operates at about 1 Hz to 3000 Hz frequencies for production of resonant mode soundwaves.

In some embodiments, signals received by a first receive channel 926 or a second receive channel 928 of the software-defined radio system 920 may be adjusted to provide a received signal gain of about -14 decibels (dB) to +60 dB.

In some embodiments, a transmitter daughterboard module is implemented to modulate an output signal to a higher frequency. In some embodiments, a receiver daughterboard module is implemented to acquire an RF signal and converts it to baseband. In some embodiments, a transceiver daughterboard module is implemented to combine the functionality of a transmitter and receiver.

In some embodiments, the FPGA of the software-defined radio system 920 performs several digital signal processing (DSP) operations. In some embodiments, the DSP operation performed by the FPGA provide translation from real signals in the analog domain to lower-rate, complex, baseband signals in the digital domain. In some embodiments, these complex samples are transferred to/from applications running on a host processor, which perform DSP operations.

In some embodiments, the hardware driver of the software-defined radio system 920 supports an operating system, such as Linux, MacOS, and Windows platforms. In some embodiments several frameworks, including GNU Radio, LabVIEW, MATLAB and Simulink may use the hardware driver. The functionality provided by hardware driver may also be accessed directly with a hardware driver application programming interface (API). In some embodiments, the API provides native support for C++ or any other language that can import C++ functions.

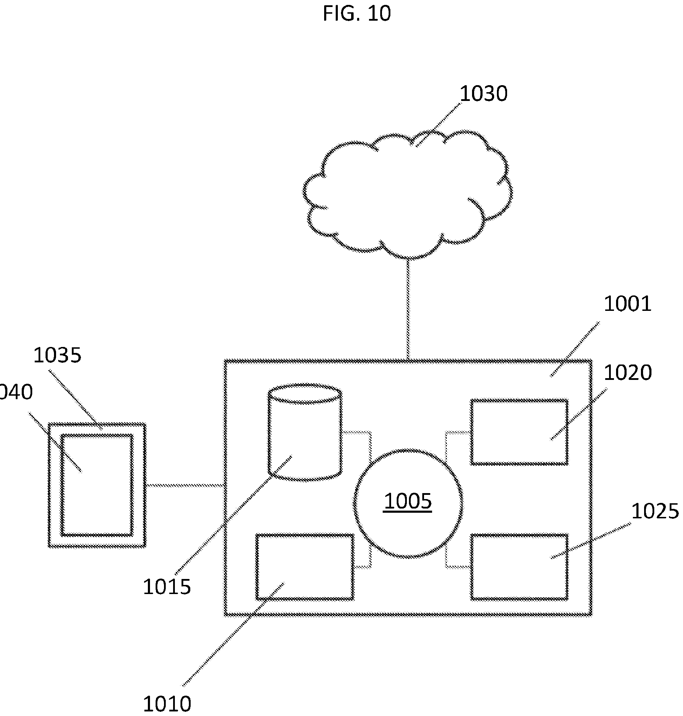

The present disclosure provides computer systems that are programmed to implement methods of the disclosure, according to some embodiments. FIG. 10 shows a computer system 1001 that is programmed or otherwise configured to, for example, implement the methods disclosed herein. The computer system 1001 can be an electronic device of a user or a computer system that is remotely located with respect to the electronic device. The electronic device can be a mobile electronic device.

The computer system 1001 includes a central processing unit (CPU, also "processor" and "computer processor" herein) 1005, which can be a single core or multi core processor, or a plurality of processors for parallel processing. The computer system 1001 also includes memory or memory location 1010 (e.g., random-access memory, read-only memory, flash memory), electronic storage unit 1015 (e.g., hard disk), communication interface 1020 (e.g., network adapter) for communicating with one or more other systems, and peripheral devices 1025, such as cache, other memory, data storage and/or electronic display adapters. The memory 1010, storage unit 1015, interface 1020 and peripheral devices 1025 are in communication with the CPU 1005 through a communication bus (solid lines), such as a motherboard. The storage unit 1015 can be a data storage unit (or data repository) for storing data. The computer system 1001 can be operatively coupled to a computer network ("network") 1030 with the aid of the communication interface 1020. The network 1030 can be the Internet, an internet and/or extranet, or an intranet and/or extranet that is in communication with the Internet. The network 1030 in some cases is a telecommunication and/or data network. The network 1030 can include one or more computer servers, which can enable distributed computing, such as cloud computing. The network 1030, in some cases with the aid of the computer system 1001, can implement a peer-to-peer network, which may enable devices coupled to the computer system 1001 to behave as a client or a server.

The CPU 1005 can execute a sequence of machine-readable instructions, which can be embodied in a program or software. The instructions may be stored in a memory location, such as the memory 1010. The instructions can be directed to the CPU 1005, which can subsequently program or otherwise configure the CPU 1005 to implement methods of the present disclosure. Examples of operations performed by the CPU 1005 can include fetch, decode, execute, and writeback.

The CPU 1005 can be part of a circuit, such as an integrated circuit. One or more other components of the system 1001 can be included in the circuit. In some cases, the circuit is an application specific integrated circuit (ASIC).

The storage unit 1015 can store files, such as drivers, libraries, and saved programs. The storage unit 1015 can store user data, e.g., user preferences and user programs. The computer system 1001 in some cases can include one or more additional data storage units that are external to the computer system 1001, such as located on a remote server that is in communication with the computer system 1001 through an intranet or the Internet.

The computer system 1001 can communicate with one or more remote computer systems through the network 1030. For instance, the computer system 1001 can communicate with a remote computer system of a user (e.g., a smartphone, a laptop computer). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system 1001 via the network 1030.

Methods as described herein can be implemented by way of machine (e.g., computer processor) executable code stored on an electronic storage location of the computer system 1001, such as, for example, on the memory 1010 or electronic storage unit 1015. The machine executable or machine-readable code can be provided in the form of software. During use, the code can be executed by the processor 1005. In some cases, the code can be retrieved from the storage unit 1015 and stored on the memory 1010 for ready access by the processor 1005. In some situations, the electronic storage unit 1015 can be precluded, and machine-executable instructions are stored on memory 1010.

The code can be pre-compiled and configured for use with a machine having a processer adapted to execute the code or can be compiled during runtime. The code can be supplied in a programming language that can be selected to enable the code to execute in a precompiled or as-compiled fashion.

Aspects of the systems and methods provided herein, such as the computer system 1001, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical, and electromagnetic waves, such as used across physical interfaces between local devices, through wired and optical landline networks and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 1001 can include or be in communication with an electronic display 1035 that comprises a user interface (UI) 1040. Examples of UI's include, without limitation, a graphical user interface (GUI), a mobile device application, and web-based user interface.

FIG. 11 illustrates a chip set 1300, according to some embodiments. Chip set 1300 can include, for instance, processor and memory components incorporated in one or more physical packages. By way of example, a physical package includes an arrangement of one or more materials, components, and/or wires on a structural assembly (e.g., a baseboard) to provide one or more characteristics such as physical strength, conservation of size, and/or limitation of electrical interaction.

In one embodiment, chip set 1300 includes a communication mechanism such as a bus 1302 for passing information among the components of the chip set 1300. A processor 1304 has connectivity to bus 1302 to execute instructions and process information stored in a memory 1306. Processor 1304 includes one or more processing cores with each core configured to perform independently. A multi-core processor enables multiprocessing within a single physical package. Examples of a multi-core processor include two, four, eight, or greater numbers of processing cores. Alternatively, or in addition, processor 1304 includes one or more microprocessors configured in tandem via bus 1302 to enable independent execution of instructions, pipelining, and multithreading. Processor 1304 may also be accompanied with one or more specialized components to perform certain processing functions and tasks such as one or more digital signal processors (DSP) 1308, and/or one or more application-specific integrated circuits (ASIC) 1310. DSP 1308 can typically be configured to process real-world signals (e.g., sound) in real time independently of processor 1304. Similarly, ASIC 1310 can be configured to performed specialized functions not easily performed by a general purposed processor. Other specialized components to aid in performing the inventive functions described herein include one or more field programmable gate arrays (FPGA) (not shown), one or more controllers (not shown), or one or more other special-purpose computer chips.

Processor 1304 and accompanying components have connectivity to the memory 1306 via bus 1302. Memory 1306 includes both dynamic memory (e.g., RAM) and static memory (e.g., ROM) for storing executable instructions that, when executed by processor 1304, DSP 1308, and/or ASIC 1310, perform the process of example embodiments as described herein. Memory 1306 also stores the data associated with or generated by the execution of the process.

In this document, the terms "machine readable medium," "computer readable medium," and similar terms are used to generally refer to non-transitory mediums, volatile or non-volatile, that store data and/or instructions that cause a machine to operate in a specific fashion. Common forms of machine readable media include, for example, a hard disk, solid state drive, magnetic tape, or any other magnetic data storage medium, an optical disc or any other optical data storage medium, any physical medium with patterns of holes, a RAM, a PROM, EPROM, a FLASH-EPROM, NVRAM, any other memory chip or cartridge, and networked versions of the same.

These and other various forms of computer readable media may be involved in carrying one or more sequences of one or more instructions to a processing device for execution. Such instructions embodied on the medium, are generally referred to as "instructions" or "code." Instructions may be grouped in the form of computer programs or other groupings. When executed, such instructions may enable a processing device to perform features or functions of the present application as discussed herein.

In this document, a "processing device" may be implemented as a single processor that performs processing operations or a combination of specialized and/or general-purpose processors that perform processing operations. A processing device may include a CPU, GPU, APU, DSP, FPGA, ASIC, SOC, and/or other processing circuitry.

The various embodiments set forth herein are described in terms of exemplary block diagrams, flow charts and other illustrations. As will become apparent to one of ordinary skill in the art after reading this document, the illustrated embodiments and their various alternatives can be implemented without confinement to the illustrated examples. For example, block diagrams and their accompanying description should not be construed as mandating a particular architecture or configuration.

Each of the processes, methods, and algorithms described in the preceding sections may be embodied in, and fully or partially automated by, code components executed by one or more computer systems or computer processors comprising computer hardware. The processes and algorithms may be implemented partially or wholly in application-specific circuitry. The various features and processes described above may be used independently of one another or may be combined in various ways. Different combinations and subcombinations are intended to fall within the scope of this disclosure, and certain method or process blocks may be omitted in some embodiments. Additionally, unless the context dictates otherwise, the methods and processes described herein are also not limited to any particular sequence, and the blocks or states relating thereto can be performed in other sequences that are appropriate, or may be performed in parallel, or in some other manner. Blocks or states may be added to or removed from the disclosed example embodiments. The performance of certain of the operations or processes may be distributed among computer systems or computers processors, not only residing within a single machine, but deployed across a number of machines.

The devices and methods described herein may be utilized for applications in fields outside of the medical fields described above. For instance, the devices and methods may be used to provide information about the internal conditions of mechanical systems, such as the engines or transmissions of vehicles. The stethoscope functionality may be used to detect abnormalities in the mechanical processes of an engine or transmission. The ultrasonic functionality may be used to image the engine or transmission to determine if it has sustained internal damage. The non-stethoscopic, non-ultrasonic sensors may provide additional information about the state of the engine or transmission, such as its temperature.

The devices and methods may be used for nondestructive testing of infrastructure. For instance, the devices and methods may be used to examine the internal structure of concrete (in streets or highways, bridges, buildings, or other structures) to determine whether the concrete or metal rebar within the concrete has been damaged. The devices and methods may be used to examine the internal structures of pipelines to determine whether they are damaged and may represent a threat to life, property, or the environment.

The devices and methods described herein may be utilized to examine the internal structures of other building materials, such as stone, brick, wood, sheetrock, thermal insulating, plastic piping, polyvinyl chloride (PVC) piping, fiberglass, or paint.

## V. CURRENTLY PREFERRED EMBODIMENT

1. In one currently preferred embodiment, the invention provides a blood pressure measurement device, comprising: a first transducer configured to emit multiple soundwaves having multiple frequencies, the soundwaves configured to cause a blood vessel of a subject to vibrate; a second transducer configured to capture one or more ultrasound images of the blood vessel; and a processing device configured to: determine, based on the one or more captured ultrasound images, a resonant frequency of the blood vessel; and calculate, based on a wall thickness of the blood vessel, a radius or diameter of the blood vessel, and the determined resonant frequency, a blood pressure of the blood vessel or the subject.

2. The blood pressure measurement device of paragraph 1, wherein the one or more captured ultrasound images are used to measure the wall thickness of the blood vessel and the radius or diameter of the blood vessel.

3. The blood pressure measurement device of paragraph 1 or 2, wherein the one or more captured ultrasound images comprise multiple ultrasound images, wherein determining the resonant frequency of the blood vessel, comprises:

determining, based on the multiple ultrasound images, a frequency of the multiple frequencies that maximized a vibration of the blood vessel; and

selecting the frequency as the resonant frequency.

4. The blood pressure measurement device of any preceding paragraph, further comprising: an audio signal generator electrically coupled to the first transducer, the audio signal generator configured to adjust a frequency of sound waves emitted by the first transducer.

5. The blood pressure measurement device of any preceding paragraph, wherein the audio signal generator comprises at least one variable resistor that adjusts the frequency of sound waves emitted by the first transducer.

6. The blood pressure measurement device of any preceding paragraph, wherein each of the frequencies is between 1 Hz and 3000 Hz.

7. The blood pressure measurement device of any preceding paragraph, wherein each of the frequencies is between 670 Hz and 2300 Hz.

8. The blood pressure measurement device of any preceding paragraph, wherein the blood vessel is a carotid artery of the subject.

9. The blood pressure measurement device of any preceding paragraph, wherein the first transducer is an audio speaker.

10. The blood pressure measurement device of any preceding paragraph, further comprising: a substrate, wherein the substrate comprises an adhesive surface for adhering to the subject's skin, wherein the first transducer and the second transducer are incorporated in the substrate.

11. The blood pressure measurement apparatus of paragraph 10, wherein the substrate is adhered proximal to the blood vessel.

12. The blood pressure measurement apparatus of paragraph 11, wherein the blood vessel is a carotid artery.

13. The blood pressure measurement apparatus of any one of paragraphs 10 to 12, wherein the substrate comprises an alignment line.

14. The blood pressure measurement apparatus of paragraph 13, wherein the substrate comprises a transparent window.

15. The blood pressure measurement device of paragraph 10, further comprising a third transducer configured to capture a second set of one or more ultrasound images of the blood vessel.

16. The blood pressure measurement device of paragraph 15, wherein the second and third transducers each have a respective resonant frequency, and wherein the second transducer comprises a frequency response that partially overlaps with a frequency response of the third transducer; and the processing device processes measurements made by the first and second transducers.

17. The blood pressure measurement device of paragraph 16, wherein the processing of measurements made by the first and second transducers comprises normalizing the first frequency response and the second frequency response.

18. The blood pressure measurement device of paragraph 15, further comprising a fourth transducer configured to capture a third set of one or more ultrasound images of the blood vessel.

19. The blood pressure measurement device of paragraph 15, further comprising: a substrate, wherein the substrate comprises an adhesive surface for adhering to the subject's skin, wherein the first transducer, the second transducer, and the third transducer are incorporated in the substrate.

20. The blood pressure measurement apparatus of paragraph 19, wherein the substrate is adhered proximal to the blood vessel.

21. The blood pressure measurement apparatus of paragraph 20, wherein the blood vessel is a carotid artery.

22. The blood pressure measurement apparatus of paragraph 21, wherein the substrate comprises an alignment line.

23. The blood pressure measurement apparatus of paragraph 22, wherein the substrate further comprises a transparent window.

24. The blood pressure measurement device of paragraph 16, further comprising a fourth transducer configured to capture a third set of one or more ultrasound images of the blood vessel.

25. The blood pressure measurement device of paragraph 24, wherein the fourth transducer comprises a frequency response that partially overlaps with the frequency response of the second transducer.

26. The blood pressure measurement device of paragraph 24, wherein the fourth transducer comprises a frequency response that partially overlaps with the frequency response of the third transducer.

27. The blood pressure measurement device of paragraph 24, wherein the fourth transducer comprises a frequency response that partially overlaps with the frequency response of the second and third transducers.

28. The blood pressure measurement device of paragraph 24, further comprising: a substrate, wherein the substrate comprises an adhesive surface for adhering to the subject's skin, wherein the first transducer, the second transducer, and the third transducer are incorporated in the substrate.

29. The blood pressure measurement apparatus of paragraph 28, wherein the substrate is adhered proximal to the blood vessel.

30. The blood pressure measurement apparatus of paragraph 29, wherein the blood vessel is a carotid artery.

31. The blood pressure measurement apparatus of paragraph 30, wherein the substrate comprises an alignment line.

32. The blood pressure measurement apparatus of paragraph 31, wherein the substrate further comprises a transparent window.

33. The blood pressure measurement device of any preceding paragraph, further comprising a third transducer configured to capture a second set of one or more ultrasound images of the blood vessel.

34. The blood pressure measurement device of paragraph 33, wherein the second and third transducers each have a respective resonant frequency, and wherein the second transducer comprises a frequency response that partially overlaps with a frequency response of the third transducer; and the processing device processes measurements made by the first and second transducers.

35. The blood pressure measurement device of paragraph 34, wherein the processing of measurements made by the first and second transducers comprises normalizing the first frequency response and the second frequency response.

36. The blood pressure measurement device of paragraph 33, further comprising a fourth transducer configured to capture a third set of one or more ultrasound images of the blood vessel.

37. The blood pressure measurement device of paragraph 34, further comprising a fourth transducer configured to capture a third set of one or more ultrasound images of the blood vessel.

38. The blood pressure measurement device of paragraph 37, wherein the fourth transducer comprises a frequency response that partially overlaps with the frequency response of the second transducer.

39. The blood pressure measurement device of paragraph 37, wherein the fourth transducer comprises a frequency response that partially overlaps with the frequency response of the third transducer.

40. The blood pressure measurement device of paragraph 37, wherein the fourth transducer comprises a frequency response that partially overlaps with the frequency response of both the second and third transducers.

41. In one currently preferred embodiment, the invention provides a non-transitory computer-readable storage medium storing instructions executable by a processor, wherein execution of the instructions causes a blood pressure measurement device to perform operations comprising: emitting, using a first transducer in proximity to a blood vessel of a subject, multiple soundwaves having multiple frequencies, the soundwaves causing a blood vessel of a subject to vibrate; determining, based on a vibrational response of the blood vessel to the multiple soundwaves, a resonant frequency of the blood vessel; determining, using a second transducer that emits ultrasonic waves, a wall thickness and a radius or diameter of the blood vessel; and calculating, based on the resonant frequency, the wall thickness of the blood vessel, and the radius or diameter of the blood vessel, a blood pressure of the subject.

42. The non-transitory computer-readable storage medium of paragraph 41, wherein the operations further comprise: capturing, using the second transducer, multiple ultrasound images of the blood vessel when it vibrates in response to the soundwaves, wherein determining the resonant frequency of the blood vessel comprises: determining the resonant frequency of the blood vessel from the ultrasound images.

43. The non-transitory computer-readable storage medium of paragraph 41 or 42, wherein determining the wall thickness and the radius of the blood vessel, comprises: directing, using the second transducer, ultrasonic waves to the blood vessel; and receiving, using the second transducer, ultrasonic waves reflected from echogenic boundaries of the blood vessel.

44. The non-transitory computer-readable storage medium of paragraph 41, wherein after calculating the blood pressure, the operations further comprise: determining, using the first transducer and second transducer, an updated radius of the blood vessel and an updated velocity of blood flowing through the blood vessel; and calculating, based on the updated radius and the updated velocity, an updated blood pressure.

45. The non-transitory computer-readable storage medium of any preceding paragraph, wherein the multiple frequencies of the multiple soundwaves are between 1 Hz and 3000 Hz.

46. The non-transitory computer-readable storage medium of any preceding paragraph, wherein the multiple frequencies of the multiple soundwaves are between 670 Hz and 2300 Hz.

47. The non-transitory computer-readable storage medium of any preceding paragraph, wherein the first transducer is an audio speaker.

48. The non-transitory computer-readable storage medium of any preceding paragraph, wherein the operations further comprise capturing, using a third transducer, a first set of ultrasound images of the blood vessel.

49. The non-transitory computer-readable storage medium of paragraph 48, wherein the operations further comprise capturing, using the second transducer, a second set of ultrasound images of the blood vessel.

50. The non-transitory computer-readable storage medium of paragraph 49, wherein the operations further comprise normalizing the second set ultrasound images by the first set of ultrasound images.

51. The non-transitory computer-readable storage medium of paragraph 49, wherein the operations further comprise capturing, using a fourth transducer, a third set of ultrasound images of the blood vessel.

52. The non-transitory computer-readable storage medium of paragraph 51, wherein the operations further comprise normalizing the second set ultrasound images by the first set of ultrasound images.

53. The non-transitory computer-readable storage medium of paragraph 52, wherein the operations further comprise normalizing the second set ultrasound images by the third set of ultrasound images.

54. In one currently preferred embodiment, the invention provides a method, comprising: emitting, using a first transducer in proximity to a blood vessel of a subject, multiple soundwaves having multiple frequencies, the soundwaves causing a blood vessel of a subject to vibrate; determining, based on the vibrational response of the blood vessel to the soundwaves, a resonant frequency of the blood vessel; determining, using a second transducer that emits ultrasonic waves, a wall thickness and a radius or diameter of the blood vessel; and calculating, based on the resonant frequency, the wall thickness of the blood vessel, and the radius or diameter of the blood vessel, a blood pressure of the subject.

55. The method of paragraph 54, further comprising: capturing, using the second transducer, multiple ultrasound images of the blood vessel when it vibrates in response to the soundwaves, wherein determining the resonant frequency of the blood vessel comprises: determining the resonant frequency of the blood vessel from the ultrasound images.

56. The method of paragraph 54 or 55, wherein determining the wall thickness and the radius of the blood vessel, comprises: directing, using the second transducer, ultrasonic waves to the blood vessel; and receiving, using the second transducer, ultrasonic waves reflected from echogenic boundaries of the blood vessel.

57. The method of any preceding paragraph, wherein after calculating the blood pressure, the method further comprises: determining, using the first transducer and second transducer, an updated radius of the blood vessel and an updated velocity of blood flowing through the blood vessel; and calculating, based on the updated radius and the updated velocity, an updated blood pressure.

58. The method of any preceding paragraph, wherein each of the frequencies is between 670 Hz and 2300 Hz.

59. The method of paragraph 55, further comprising capturing, using a third transducer, a second set of multiple ultrasound images of the blood vessel when it vibrates in response to the soundwaves.

60. The method of paragraph 59, further comprising normalizing the multiple ultrasound images of the blood vessel captured by the second transducer with the second set of multiple ultrasound images of the blood vessel captured by the third transducer.

61. The method of paragraph 59, further comprising capturing, using a fourth transducer, a third set of multiple ultrasound images of the blood vessel when it vibrates in response to the soundwaves.

62. The method of paragraph 61, further comprising normalizing the multiple ultrasound images of the blood vessel captured by the second transducer with the second set of multiple ultrasound images of the blood vessel captured by the third transducer.

63. The method of paragraph 62, further comprising normalizing the multiple ultrasound images of the blood vessel captured by the second transducer with the third set of multiple ultrasound images of the blood vessel captured by the fourth transducer.

64. In one currently preferred embodiment, the invention provides a blood pressure measurement apparatus comprising: a first transducer configured to direct sound waves to a blood vessel; and a second transducer configured to direct ultrasonic waves to the blood vessel, receive ultrasonic waves reflected by echogenic boundaries of the blood vessel, measure a or diameter radius of a cross-section of the blood vessel and measure a wall thickness of the cross section of the blood vessel.

65. The blood pressure measurement apparatus of paragraph 64, wherein the first transducer is an electroacoustic transducer and the second transducer is a piezoelectric ultrasonic transducer.

66. The blood pressure measurement apparatus of paragraph 64 or 65, wherein the apparatus further comprises an audio signal generator coupled to the first transducer.

67. The blood pressure measurement apparatus of paragraph 66, wherein the audio signal generator is configured to vary a frequency of the sound waves.

68. The blood pressure measurement apparatus of paragraph 67, further comprising a display.

69. The blood pressure measurement apparatus of paragraphs 68, wherein the display shows the frequency of the sound waves.

70. The blood pressure measurement apparatus of paragraph 69 wherein the second transducer monitors a vibration of the cross-section of the blood vessel.

71. The blood pressure measurement apparatus of paragraph 67 wherein the second transducer monitors a vibration of the cross-section of the blood vessel, and wherein the second transducer records the frequency of the vibration of the cross-section and determines a resonant frequency thereof.

72. The blood pressure measurement apparatus of paragraph 71, wherein the resonant frequency is determined when the vibration of the cross-section of the blood vessel is at a maximum

73. The blood pressure measurement apparatus of any preceding paragraph, wherein a frequency of the sound waves is varied over a range of 1 Hz to 3000 Hz.

74. The blood pressure measurement apparatus of any preceding paragraph, wherein a frequency of the sound waves is varied over a range of 670 Hz to 2300 Hz.

75. The blood pressure measurement apparatus of any preceding paragraph, wherein the electroacoustic transducer is an audio speaker.

76. The blood pressure measurement apparatus of paragraph 75, wherein the audio speaker is a tweeter.

77. The blood pressure measurement apparatus of any one of paragraphs 64 to 76, wherein the blood vessel is an artery or a vein.

78. The blood pressure measurement apparatus of any preceding paragraph, wherein the first and second transducers are coupled to a substrate, and wherein the substrate comprises an adhesive backing.

79. The blood pressure measurement apparatus of paragraph 78, wherein the substrate is adhered proximal to the blood vessel.

80. The blood pressure measurement apparatus of paragraph 79, wherein the blood vessel is a carotid artery.

81. The blood pressure measurement apparatus of any one of paragraphs 78 to 80, wherein the substrate comprises an alignment line.

82. The blood pressure measurement apparatus of any one of paragraphs 78 to 80, wherein the substrate comprises an alignment line and a transparent window.

83. In one currently preferred embodiment, the invention provides a method of measuring blood pressure comprising: determining a radius and wall thickness of a cross section of a blood vessel; directing sound waves to the blood vessel; varying a frequency of the sound waves; detecting a maximum resonance of the cross-section of the blood vessel to determine a resonant frequency of the blood vessel; and calculating, based on the determined resonant frequency, radius, and wall thickness of the blood, a blood pressure in the blood vessel.

84. The method of paragraph 83, wherein varying the frequency of the sound waves comprises varying the frequency of the sound waves through a range of 1 Hz to 3000 Hz.

85. The method of paragraph 83, wherein varying the frequency of the sound waves comprises varying the frequency of the sound waves through a range of 670 Hz to 2300 Hz.

86. The method of any preceding paragraph, wherein the resonant frequency is determined by use of a piezoelectric ultrasonic transducer for detecting the maximum resonance.

87. The method of paragraph 86, wherein the piezoelectric ultrasonic transducer comprises a sampling rate of at least 3 kHz.

88. The method of any preceding paragraph, wherein the step of determining the radius and wall thickness of the blood vessel comprises directing ultrasonic waves to the blood vessel and receiving reflected ultrasonic waves reflected from echogenic boundaries of the blood vessel.

89. The method of paragraph 88, further comprising measuring a doppler shift of the reflected ultrasonic waves, and calculating a wave velocity of the blood vessel.

90. The method of paragraph 89, wherein the doppler shift is measured by a piezoelectric ultrasonic transducer.

91. The method of any preceding paragraph, wherein the blood vessel is an artery or a vein.

92. The method of any preceding paragraph, further comprising directing a first set of transmitted ultrasound waves toward the blood vessel and capturing a first set of reflected ultrasound waves from the blood vessel.

93. The method of paragraph 92, wherein the steps of directing a first set of transmitted ultrasound waves toward the blood vessel and capturing a first set of reflected ultrasound waves from the blood vessel are conducted by a single ultrasound transducer.

94. The method of any preceding paragraph, further comprising directing, with a first ultrasound transducer, a first set of transmitted ultrasound waves toward the blood vessel and capturing, with a second ultrasound transducer, a first set of reflected ultrasound waves from the blood vessel.

95. The method of any preceding paragraph, further comprising directing, with a first ultrasound transducer, a first set of transmitted ultrasound waves toward the blood vessel and capturing, with the first ultrasound transducer and a second ultrasound, a first set of reflected ultrasound waves from the blood vessel.

96. The method of any preceding paragraph, further comprising directing, with a first ultrasound transducer, a first set of transmitted ultrasound signal toward the blood vessel and capturing, with the first ultrasound transducer, a first reflected ultrasound signal from the blood vessel.

97. The method of paragraph 96, further capturing, with a second ultrasound transducer, a second reflected ultrasound signal from the blood vessel.

98. The method of paragraph 97, further comprising normalizing the first reflected ultrasound signal with the second reflected ultrasound signal.

99. The method of paragraph 97, further comprising capturing, with a third ultrasound transducer, a third reflected ultrasound signal from the blood vessel.

100. The method of paragraph 99, further comprising normalizing the first reflected ultrasound signal with the third reflected ultrasound signal.

101. The method of any preceding paragraph, wherein the blood vessel is carotid artery.

102. In one currently preferred embodiment, the invention provides a system for transmitting and receiving ultrasound signals comprising: a software-defined radio comprising one or more outputs and one or more inputs; an ultrasound signal processing circuit electrically coupled to the software-defined radio; and one or more ultrasound transducers electrically coupled to the ultrasound signal processing circuit, wherein the ultrasound signal processing circuit processes one or more ultrasound transmission signals from the one or more outputs of the software-defined radio and transmits processed ultrasound transmission signals to the one or more ultrasound transducers to produce ultrasound waves, and wherein the signal processing unit processes one or more received ultrasound signals from the one or more ultrasound transducers and transmits processed received ultrasound signals to the one or more inputs of the software-defined radio.

103. The system of paragraph 102, wherein the ultrasound processing circuit comprises at least one high voltage amplifier to amplify the one or more ultrasound transmission signals from the one or more outputs of the software-defined radio.

104. The system of paragraph 103, wherein the ultrasound processing circuit comprises at least one variable gain amplifier to amplify the one or more received ultrasound signals from the one or more ultrasound transducers.

105. The system of any preceding paragraph, wherein the ultrasound processing circuit comprises at least one variable gain amplifier to amplify the one or more received ultrasound signals from the one or more ultrasound transducers.

106. The system of any preceding paragraph, wherein the outputs of the software-defined radio comprise a gain ramp and a transmission pulse.

107. The system of paragraph 106, wherein the ultrasound processing circuit comprises a high voltage amplifier to amplify the transmission pulse.

108. The system of paragraph 107, wherein the ultrasound processing circuit comprises a variable gain amplifier to amplify the one or more received ultrasound signals from the one or more ultrasound transducers based on the gain ramp transmitted by the software-defined radio.

109. The system of paragraph 108, wherein a rate of amplification of the ramp signal corresponds to the time since an initial pulse of the ramp signal was emitted by the software-defined radio.

110. The system of paragraph 109, the ultrasound transducers comprise a first pixel group and a second pixel group.

111. The system of paragraph 110, wherein the first pixel group and the second pixel group each comprise 16 pixels.

112. The system of paragraph 111, wherein the first pixel group is configured to receive the processed ultrasound transmission signals.

113. The system of paragraph 111, wherein only the first pixel group receives the processed ultrasound transmission signals.

114. The system of paragraph 113, wherein the one or more received ultrasound signals are received transmitted to the ultrasound processing circuit by the first pixel group and the second pixel group.

115. The system of paragraph 108, wherein the variable gain amplifier is a low noise, single-ended, linear, general purpose variable gain amplifier.

116. The system of any preceding paragraph, wherein the ultrasound transducers comprise a first pixel group and a second pixel group.

117. The system of paragraph 116, wherein the first pixel group is configured to receive the processed ultrasound transmission signals.

118. The system of paragraph 117, wherein only the first pixel group receives the processed ultrasound transmission signals.

119. The system of paragraph 118, further comprising a computing device connected to the software-defined radio, such that the computing device controls the one or more outputs of the software-defined radio.

120. The system of paragraph 119, wherein the computing device comprises a display, wherein the display displays data received by the one or more inputs of the software-defined radio.

121. The system of paragraph 120, wherein the display displays one or more ultrasonic images obtained by the one or more ultrasound transducers.

122. The system of any preceding paragraph, further comprising a computing device connected to the software-defined radio, such that the computing device controls the one or more outputs of the software-defined radio.

123. The system of paragraph 122, wherein the computing device comprises a display, wherein the display displays data received by the one or more inputs of the software-defined radio.

124. The system of paragraph 123, wherein the display displays one or more ultrasonic images obtained by the one or more ultrasound transducers.

125. In one currently preferred embodiment, the invention provides a method of conditioning one or more ultrasound signals comprising: receiving a first transmission signal from a software-defined radio; amplifying the first transmission signal, thereby forming an amplified transmission signal; forwarding, with a first multiplexer, the amplified transmission signal to one or more ultrasound transducers; receiving, with the first multiplexer, one or more received ultrasound signals from the one or more ultrasound transducers; and amplifying the one or more received ultrasound signals, thereby forming one or more amplified received ultrasound signals.

126. The method of any preceding paragraph, wherein amplification of the one or more received ultrasound signals is based on a gain ramp transmitted by the software-defined radio.

127. The method of paragraph 126, wherein the amplification is time dependent.

128. The method of paragraph 127, wherein amplification of the one or more received ultrasound signals is carried out by one or more low noise, single-ended, linear, general purpose variable gain amplifiers.

129. The method of any preceding paragraph, wherein amplification of the one or more received ultrasound signals is carried out by one or more low noise, single-ended, linear, general purpose variable gain amplifiers.

130. The method of any preceding paragraph, further comprising emitting one or more ultrasound waves toward at least one object, wherein the at least one object reflects the one or more ultrasound waves, and wherein the reflected ultrasound waves are detected by the one or more ultrasound transducers and form the one or more received ultrasound signals.

131. The method of paragraph 130, wherein amplification of the one or more received ultrasound signals is based on a gain ramp transmitted by the software-defined radio.

132. The method of paragraph 131, wherein the amplification is time dependent.

133. The method of paragraph 132, wherein amplification of the one or more received ultrasound signals is carried out by one or more low noise, single-ended, linear, general purpose variable gain amplifiers.

134. The method of paragraph 130, wherein the at least one object is a blood vessel of a subject.

135. The method of paragraph 134, wherein the blood vessel is a carotid artery.

## VI. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Throughout this application, various embodiments may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used in the specification and claims, the singular forms "a", "an" and "the" include plural references unless the context clearly dictates otherwise. For example, the term "a sample" includes a plurality of samples, including mixtures thereof.

The terms "determining," "measuring," "evaluating," "assessing," "assaying," and "analyzing" are often used interchangeably herein to refer to forms of measurement. The terms include determining if an element is present or not (for example, detection). These terms can include quantitative, qualitative or quantitative and qualitative determinations. Assessing can be relative or absolute. "Detecting the presence of" can include determining the amount of something present in addition to determining whether it is present or absent depending on the context.

The terms "subject," "individual," or "patient" are often used interchangeably herein. A "subject" can be a biological entity containing expressed genetic materials. The biological entity can be a plant, animal, or microorganism, including, for example, bacteria, viruses, fungi, and protozoa. The subject can be tissues, cells and their progeny of a biological entity obtained in vivo or cultured in vitro. The subject can be a mammal. The mammal can be a human. The subject may be diagnosed or suspected of being at high risk for a disease. In some cases, the subject is not necessarily diagnosed or suspected of being at high risk for the disease.

The term *"in vivo"* is used to describe an event that takes place in a subject's body.

The term *"ex vivo"* is used to describe an event that takes place outside of a subject's body. An *ex vivo* assay is not performed on a subject. Rather, it is performed upon a sample separate from a subject. An example of an *ex vivo* assay performed on a sample is an *"in vitro"* assay.

The term *"in vitro"* is used to describe an event that takes places contained in a container for holding laboratory reagent such that it is separated from the biological source from which the material is obtained. *In vitro* assays can encompass cell-based assays in which living or dead cells are employed. *In vitro* assays can also encompass a cell-free assay in which no intact cells are employed.

As used herein, the term "about" a number refers to that number plus or minus 10% of that number. The term "about" a range refers to that range minus 10% of its lowest value and plus 10% of its greatest value.

As used herein, the term "or" may be construed in either an inclusive or exclusive sense. Moreover, the description of resources, operations, or structures in the singular shall not be read to exclude the plural. Conditional language, such as, among others, "can," "could," "might," or "may," unless specifically stated otherwise, or otherwise understood within the context as used, is generally intended to convey that certain embodiments include, while other embodiments do not include, certain features, elements and/or steps.

Terms and phrases used in this document, and variations thereof, unless otherwise expressly stated, should be construed as open ended as opposed to limiting. Adjectives such as "conventional," "traditional," "normal," "standard," "known," and terms of similar meaning should not be construed as limiting the item described to a given time period or to an item available as of a given time, but instead should be read to encompass conventional, traditional, normal, or standard technologies that may be available or known now or at any time in the future. The presence of broadening words and phrases such as "one or more," "at least," "but not limited to" or other like phrases in some instances shall not be read to mean that the narrower case is intended or required in instances where such broadening phrases may be absent.

The section headings used herein are for organizational purposes only and are not to be construed as limiting the subject matter described.

## VII. EXAMPLES

The following examples are included for illustrative purposes only and are not intended to limit the scope of the invention.

### Example 1: Measurement of Blood Pressure Using a Cuff

In an example of measuring blood pressure using previous methods which utilize a sphygmomanometer cuff, a patient's arm is laid out on a comfortable surface. A trained nurse or doctor then places a properly sized cuff around the upper arm of the patient. A stethoscope is placed on the brachial artery of the patient just below the cuff.

The trained person then inflates the cuff to 180 mmHG and begins releasing air from the cuff at a suggest rate of 3mm/sec. The trained person must simultaneously observe they sphygmomanometer of the cuff while listening with the stethoscope.

A first "knocking sound" indicates the point at which the systolic pressure of the patient is to be measured using the sphygmomanometer. When the "knocking sound" disappears, the diastolic pressure of the patient is to be measured using the sphygmomanometer. The measured systolic and diastolic pressures should be recorded.

It is further recommended that measurements are taken in the other arm. Any difference between measurements of the arm should be recorded. Further, the subject's position and the cuff sized used should be recorded.

If the subject's blood pressure appears to be elevated, it is recommended that the patient's blood pressure be measured at least two additional times. With a rest period in given in between measurements.

### Example 2: Measurement of Blood Pressure using the Arterial Resonance

An adhesive substrate comprising one or more ultrasound transducers and an electroacoustic transducer is adhered proximal to the patient's carotid artery. An electroacoustic transducer provided on the adhesive substrate is activated.

The electroacoustic transducer is varied through a range of audio frequencies. The ultrasound transducer detects when resonance in the artery is at a maximum level. The resonance frequency is held for a moment and recorded. The ultrasound transducer determines the arterial wall thickness and diameter of the artery. The blood pressure of the patient is then calculated and recorded.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

Further embodiments are set out in the following clauses:

1. A blood pressure measurement device, comprising: a first transducer configured to emit multiple soundwaves having multiple frequencies, the soundwaves configured to cause a blood vessel of a subject to vibrate; a second transducer configured to capture one or more ultrasound images of the blood vessel; and a processing device configured to: determine, based on the one or more captured ultrasound images, a resonant frequency of the blood vessel; and calculate, based on a wall thickness of the blood vessel, a radius or diameter of the blood vessel, and the determined resonant frequency, a blood pressure of the blood vessel or the subject.

2. The blood pressure measurement device of clause 1, wherein the one or more captured ultrasound images are used to measure the wall thickness of the blood vessel and the radius or diameter of the blood vessel.

3. The blood pressure measurement device of clause 1, wherein the one or more captured ultrasound images comprise multiple ultrasound images, wherein determining the resonant frequency of the blood vessel, comprises: determining, based on the multiple ultrasound images, a frequency of the multiple frequencies that maximized a vibration of the blood vessel; and selecting the frequency as the resonant frequency.

4. The blood pressure measurement device of clause 1, further comprising: an audio signal generator electrically coupled to the first transducer, the audio signal generator configured to adjust a frequency of sound waves emitted by the first transducer.

5. The blood pressure measurement device of clause 1, wherein the audio signal generator comprises at least one variable resistor that adjusts the frequency of sound waves emitted by the first transducer.

6. The blood pressure measurement device of clause 1, wherein each of the frequencies is between 1 Hz and 3000 Hz.

7. The blood pressure measurement device of clause 6, wherein each of the frequencies is between 670 Hz and 2300 Hz.

8. The blood pressure measurement device of clause 1, wherein the blood vessel is a carotid artery of the subject.

9. The blood pressure measurement device of clause 1, wherein the first transducer is an audio speaker.

10. The blood pressure measurement device of clause 1, further comprising: a substrate, wherein the substrate comprises an adhesive surface for adhering to the subject's skin, wherein the first transducer and the second transducer are incorporated in the substrate.

11. The blood pressure measurement apparatus of clause 10, wherein the substrate is adhered proximal to the blood vessel.

12. The blood pressure measurement apparatus of clause 11, wherein the blood vessel is a carotid artery.

13. The blood pressure measurement apparatus of any one of clauses 10 to 12, wherein the substrate comprises an alignment line.

14. The blood pressure measurement apparatus of clause 13, wherein the substrate comprises a transparent window.

15. The blood pressure measurement device of clause 10, further comprising a third transducer configured to capture a second set of one or more ultrasound images of the blood vessel.

16. The blood pressure measurement device of clause 15, wherein the second and third transducers each have a respective resonant frequency, and wherein the second transducer comprises a frequency response that partially overlaps with a frequency response of the third transducer; and the processing device processes measurements made by the first and second transducers.

17. The blood pressure measurement device of clause 16, wherein the processing of measurements made by the first and second transducers comprises normalizing the first frequency response and the second frequency response.

18. The blood pressure measurement device of clause 15, further comprising a fourth transducer configured to capture a third set of one or more ultrasound images of the blood vessel.

19. The blood pressure measurement device of clause 15, further comprising: a substrate, wherein the substrate comprises an adhesive surface for adhering to the subject's skin, wherein the first transducer, the second transducer, and the third transducer are incorporated in the substrate.

20. The blood pressure measurement apparatus of clause 19, wherein the substrate is adhered proximal to the blood vessel.

21. The blood pressure measurement apparatus of clause 20, wherein the blood vessel is a carotid artery.

22. The blood pressure measurement apparatus of clause 21, wherein the substrate comprises an alignment line.

23. The blood pressure measurement apparatus of clause 22, wherein the substrate further comprises a transparent window.

24. The blood pressure measurement device of clause 16, further comprising a fourth transducer configured to capture a third set of one or more ultrasound images of the blood vessel.

25. The blood pressure measurement device of clause 24, wherein the fourth transducer comprises a frequency response that partially overlaps with the frequency response of the second transducer.

26. The blood pressure measurement device of clause 24, wherein the fourth transducer comprises a frequency response that partially overlaps with the frequency response of the third transducer.

27. The blood pressure measurement device of clause 24, wherein the fourth transducer comprises a frequency response that partially overlaps with the frequency response of the second and third transducers.

28. The blood pressure measurement device of clause 24, further comprising: a substrate, wherein the substrate comprises an adhesive surface for adhering to the subject's skin, wherein the first transducer, the second transducer, and the third transducer are incorporated in the substrate.

29. The blood pressure measurement apparatus of clause 28, wherein the substrate is adhered proximal to the blood vessel.

30. The blood pressure measurement apparatus of clause 29, wherein the blood vessel is a carotid artery.

31. The blood pressure measurement apparatus of clause 30, wherein the substrate comprises an alignment line.

32. The blood pressure measurement apparatus of clause 31, wherein the substrate further comprises a transparent window.

33. The blood pressure measurement device of clause 1, further comprising a third transducer configured to capture a second set of one or more ultrasound images of the blood vessel.

34. The blood pressure measurement device of clause 33, wherein the second and third transducers each have a respective resonant frequency, and wherein the second transducer comprises a frequency response that partially overlaps with a frequency response of the third transducer; and the processing device processes measurements made by the first and second transducers.

35. The blood pressure measurement device of clause 34, wherein the processing of measurements made by the first and second transducers comprises normalizing the first frequency response and the second frequency response.

36. The blood pressure measurement device of clause 33, further comprising a fourth transducer configured to capture a third set of one or more ultrasound images of the blood vessel.

37. The blood pressure measurement device of clause 34, further comprising a fourth transducer configured to capture a third set of one or more ultrasound images of the blood vessel.

38. The blood pressure measurement device of clause 37, wherein the fourth transducer comprises a frequency response that partially overlaps with the frequency response of the second transducer.

39. The blood pressure measurement device of clause 37, wherein the fourth transducer comprises a frequency response that partially overlaps with the frequency response of the third transducer.

40. The blood pressure measurement device of clause 37, wherein the fourth transducer comprises a frequency response that partially overlaps with the frequency response of both the second and third transducers.

41. A non-transitory computer-readable storage medium storing instructions executable by a processor, wherein execution of the instructions causes a blood pressure measurement device to perform operations comprising: emitting, using a first transducer in proximity to a blood vessel of a subject, multiple soundwaves having multiple frequencies, the soundwaves causing a blood vessel of a subject to vibrate; determining, based on a vibrational response of the blood vessel to the multiple soundwaves, a resonant frequency of the blood vessel; determining, using a second transducer that emits ultrasonic waves, a wall thickness and a radius or diameter of the blood vessel; and calculating, based on the resonant frequency, the wall thickness of the blood vessel, and the radius or diameter of the blood vessel, a blood pressure of the subject.

42. The non-transitory computer-readable storage medium of clause 41, wherein the operations further comprise: capturing, using the second transducer, multiple ultrasound images of the blood vessel when it vibrates in response to the soundwaves, wherein determining the resonant frequency of the blood vessel comprises: determining the resonant frequency of the blood vessel from the ultrasound images.

43. The non-transitory computer-readable storage medium of clause 41, wherein determining the wall thickness and the radius of the blood vessel, comprises: directing, using the second transducer, ultrasonic waves to the blood vessel; and receiving, using the second transducer, ultrasonic waves reflected from echogenic boundaries of the blood vessel.

44. The non-transitory computer-readable storage medium of clause 41, wherein after calculating the blood pressure, the operations further comprise:

determining, using the first transducer and second transducer, an updated radius of the blood vessel and an updated velocity of blood flowing through the blood vessel; and calculating, based on the updated radius and the updated velocity, an updated blood pressure.

45. The non-transitory computer-readable storage medium of clause 41, wherein the multiple frequencies of the multiple soundwaves are between 1 Hz and 3000 Hz.

46. The non-transitory computer-readable storage medium of clause 41, wherein the multiple frequencies of the multiple soundwaves are between 670 Hz and 2300 Hz.

47. The non-transitory computer-readable storage medium of clause 41, wherein the first transducer is an audio speaker.

48. The non-transitory computer-readable storage medium of clause 41, wherein the operations further comprise capturing, using a third transducer, a first set of ultrasound images of the blood vessel.

49. The non-transitory computer-readable storage medium of clause 48, wherein the operations further comprise capturing, using the second transducer, a second set of ultrasound images of the blood vessel.

50. The non-transitory computer-readable storage medium of clause 49, wherein the operations further comprise normalizing the second set ultrasound images by the first set of ultrasound images.

51. The non-transitory computer-readable storage medium of clause 49, wherein the operations further comprise capturing, using a fourth transducer, a third set of ultrasound images of the blood vessel.

52. The non-transitory computer-readable storage medium of clause 51, wherein the operations further comprise normalizing the second set ultrasound images by the first set of ultrasound images.

53. The non-transitory computer-readable storage medium of clause 52, wherein the operations further comprise normalizing the second set ultrasound images by the third set of ultrasound images.

54. A method, comprising: emitting, using a first transducer in proximity to a blood vessel of a subject, multiple soundwaves having multiple frequencies, the soundwaves causing a blood vessel of a subject to vibrate; determining, based on the vibrational response of the blood vessel to the soundwaves, a resonant frequency of the blood vessel; determining, using a second transducer that emits ultrasonic waves, a wall thickness and a radius or diameter of the blood vessel; and calculating, based on the resonant frequency, the wall thickness of the blood vessel, and the radius or diameter of the blood vessel, a blood pressure of the subject.

55. The method of clause 54, further comprising: capturing, using the second transducer, multiple ultrasound images of the blood vessel when it vibrates in response to the soundwaves, wherein determining the resonant frequency of the blood vessel comprises: determining the resonant frequency of the blood vessel from the ultrasound images.

56. The method of clause 54, wherein determining the wall thickness and the radius of the blood vessel, comprises: directing, using the second transducer, ultrasonic waves to the blood vessel; and receiving, using the second transducer, ultrasonic waves reflected from echogenic boundaries of the blood vessel.

57. The method of clause 54, wherein after calculating the blood pressure, the method further comprises: determining, using the first transducer and second transducer, an updated radius of the blood vessel and an updated velocity of blood flowing through the blood vessel; and calculating, based on the updated radius and the updated velocity, an updated blood pressure.

58. The method of clause 54, wherein each of the frequencies is between 670 Hz and 2300 Hz.

59. The method of clause 55, further comprising capturing, using a third transducer, a second set of multiple ultrasound images of the blood vessel when it vibrates in response to the soundwaves.

60. The method of clause 59, further comprising normalizing the multiple ultrasound images of the blood vessel captured by the second transducer with the second set of multiple ultrasound images of the blood vessel captured by the third transducer.

61. The method of clause 59 , further comprising capturing, using a fourth transducer, a third set of multiple ultrasound images of the blood vessel when it vibrates in response to the soundwaves.

62. The method of clause 61, further comprising normalizing the multiple ultrasound images of the blood vessel captured by the second transducer with the second set of multiple ultrasound images of the blood vessel captured by the third transducer.

63. The method of clause 62, further comprising normalizing the multiple ultrasound images of the blood vessel captured by the second transducer with the third set of multiple ultrasound images of the blood vessel captured by the fourth transducer.

64. A blood pressure measurement apparatus comprising: a first transducer configured to direct sound waves to a blood vessel; and a second transducer configured to direct ultrasonic waves to the blood vessel, receive ultrasonic waves reflected by echogenic boundaries of the blood vessel, measure a or diameter radius of a cross-section of the blood vessel, and measure a wall thickness of the cross section of the blood vessel.

65. The blood pressure measurement apparatus of clause 64, wherein the first transducer is an electroacoustic transducer and the second transducer is a piezoelectric ultrasonic transducer.

66. The blood pressure measurement apparatus of clause 65, wherein the apparatus further comprises an audio signal generator coupled to the first transducer.

67. The blood pressure measurement apparatus of clause 66, wherein the audio signal generator is configured to vary a frequency of the sound waves.

68. The blood pressure measurement apparatus of clause 67, further comprising a display.

69. The blood pressure measurement apparatus of clauses 68, wherein the display shows the frequency of the sound waves.

70. The blood pressure measurement apparatus of clause 69 wherein the second transducer monitors a vibration of the cross-section of the blood vessel.

71. The blood pressure measurement apparatus of clause 67 wherein the second transducer monitors a vibration of the cross-section of the blood vessel, and wherein the second transducer records the frequency of the vibration of the cross-section and determines a resonant frequency thereof.

72. The blood pressure measurement apparatus of clause 71, wherein the resonant frequency is determined when the vibration of the cross-section of the blood vessel is at a maximum

73. The blood pressure measurement apparatus of clause 64, wherein a frequency of the sound waves is varied over a range of 1 Hz to 3000 Hz.

74. The blood pressure measurement apparatus of clause 73, wherein a frequency of the sound waves is varied over a range of 670 Hz to 2300 Hz.

75. The blood pressure measurement apparatus of clause 65, wherein the electroacoustic transducer is an audio speaker.

76. The blood pressure measurement apparatus of clause 75, wherein the audio speaker is a tweeter.

77. The blood pressure measurement apparatus of any one of clauses 64 to 76, wherein the blood vessel is an artery or a vein.

78. The blood pressure measurement apparatus of clause 64, wherein the first and second transducers are coupled to a substrate, and wherein the substrate comprises an adhesive backing.

79. The blood pressure measurement apparatus of clause 78, wherein the substrate is adhered proximal to the blood vessel.

80. The blood pressure measurement apparatus of clause 79, wherein the blood vessel is a carotid artery.

81. The blood pressure measurement apparatus of any one of clauses 78 to 80, wherein the substrate comprises an alignment line.

82. The blood pressure measurement apparatus of any one of clauses 78 to 80, wherein the substrate comprises an alignment line and a transparent window.

83. A method of measuring blood pressure comprising: determining a radius and wall thickness of a cross section of a blood vessel; directing sound waves to the blood vessel; varying a frequency of the sound waves; detecting a maximum resonance of the cross-section of the blood vessel to determine a resonant frequency of the blood vessel; and calculating, based on the determined resonant frequency, radius, and wall thickness of the blood, a blood pressure in the blood vessel.

84. The method of clause 83, wherein varying the frequency of the sound waves comprises varying the frequency of the sound waves through a range of 1 Hz to 3000 Hz.

85. The method of clause 83, wherein varying the frequency of the sound waves comprises varying the frequency of the sound waves through a range of 670 Hz to 2300 Hz.

86. The method of clause 83, wherein the resonant frequency is determined by use of a piezoelectric ultrasonic transducer for detecting the maximum resonance.

87. The method of clause 86, wherein the piezoelectric ultrasonic transducer comprises a sampling rate of at least 3 kHz.

88. The method of clause 83, wherein the step of determining the radius and wall thickness of the blood vessel comprises directing ultrasonic waves to the blood vessel and receiving reflected ultrasonic waves reflected from echogenic boundaries of the blood vessel.

89. The method of clause 88, further comprising measuring a doppler shift of the reflected ultrasonic waves, and calculating a wave velocity of the blood vessel.

90. The method of clause 89, wherein the doppler shift is measured by a piezoelectric ultrasonic transducer.

91. The method of any one of clauses 83 to 90, wherein the blood vessel is an artery or a vein.

92. The method of clause 83, further comprising directing a first set of transmitted ultrasound waves toward the blood vessel and capturing a first set of reflected ultrasound waves from the blood vessel.

93. The method of clause 92, wherein the steps of directing a first set of transmitted ultrasound waves toward the blood vessel and capturing a first set of reflected ultrasound waves from the blood vessel are conducted by a single ultrasound transducer.

94. The method of clause 83, further comprising directing, with a first ultrasound transducer, a first set of transmitted ultrasound waves toward the blood vessel and capturing, with a second ultrasound transducer, a first set of reflected ultrasound waves from the blood vessel.

95. The method of clause 83, further comprising directing, with a first ultrasound transducer, a first set of transmitted ultrasound waves toward the blood vessel and capturing, with the first ultrasound transducer and a second ultrasound, a first set of reflected ultrasound waves from the blood vessel.

96. The method of clause 83, further comprising directing, with a first ultrasound transducer, a first set of transmitted ultrasound signal toward the blood vessel and capturing, with the first ultrasound transducer, a first reflected ultrasound signal from the blood vessel.

97. The method of clause 96, further capturing, with a second ultrasound transducer, a second reflected ultrasound signal from the blood vessel.

98. The method of clause 97, further comprising normalizing the first reflected ultrasound signal with the second reflected ultrasound signal.

99. The method of clause 97, further comprising capturing, with a third ultrasound transducer, a third reflected ultrasound signal from the blood vessel.

100. The method of clause 99, further comprising normalizing the first reflected ultrasound signal with the third reflected ultrasound signal.

101. The method of clause 83, wherein the blood vessel is carotid artery.

102. A system for transmitting and receiving ultrasound signals comprising: a software-defined radio comprising one or more outputs and one or more inputs; an ultrasound signal processing circuit electrically coupled to the software-defined radio; and one or more ultrasound transducers electrically coupled to the ultrasound signal processing circuit, wherein the ultrasound signal processing circuit processes one or more ultrasound transmission signals from the one or more outputs of the software-defined radio and transmits processed ultrasound transmission signals to the one or more ultrasound transducers to produce ultrasound waves, and wherein the signal processing unit processes one or more received ultrasound signals from the one or more ultrasound transducers and transmits processed received ultrasound signals to the one or more inputs of the software-defined radio.

103. The system of clause 102, wherein the ultrasound processing circuit comprises at least one high voltage amplifier to amplify the one or more ultrasound transmission signals from the one or more outputs of the software-defined radio.

104. The system of clause 103, wherein the ultrasound processing circuit comprises at least one variable gain amplifier to amplify the one or more received ultrasound signals from the one or more ultrasound transducers.

105. The system of clause 102, wherein the ultrasound processing circuit comprises at least one variable gain amplifier to amplify the one or more received ultrasound signals from the one or more ultrasound transducers.

106. The system of clause 102, wherein the outputs of the software-defined radio comprise a gain ramp and a transmission pulse.

107. The system of clause 106, wherein the ultrasound processing circuit comprises a high voltage amplifier to amplify the transmission pulse.

108. The system of clause 107, wherein the ultrasound processing circuit comprises a variable gain amplifier to amplify the one or more received ultrasound signals from the one or more ultrasound transducers based on the gain ramp transmitted by the software-defined radio.

109. The system of clause 108, wherein a rate of amplification of the ramp signal corresponds to the time since an initial pulse of the ramp signal was emitted by the software-defined radio.

110. The system of clause 109, the ultrasound transducers comprise a first pixel group and a second pixel group.

111. The system of clause 110, wherein the first pixel group and the second pixel group each comprise 16 pixels.

112. The system of clause 111, wherein the first pixel group is configured to receive the processed ultrasound transmission signals.

113. The system of clause 111, wherein only the first pixel group receives the processed ultrasound transmission signals.

114. The system of clause 113, wherein the one or more received ultrasound signals are received transmitted to the ultrasound processing circuit by the first pixel group and the second pixel group.

115. The system of clause 108, wherein the variable gain amplifier is a low noise, single- ended, linear, general purpose variable gain amplifier.

116. The system of clause 102, wherein the ultrasound transducers comprise a first pixel group and a second pixel group.

117. The system of clause 116, wherein the first pixel group is configured to receive the processed ultrasound transmission signals.

118. The system of clause 117, wherein only the first pixel group receives the processed ultrasound transmission signals.

119. The system of clause 118, further comprising a computing device connected to the software-defined radio, such that the computing device controls the one or more outputs of the software-defined radio.

120. The system of clause 119, wherein the computing device comprises a display, wherein the display displays data received by the one or more inputs of the software-defined radio.

121. The system of clause 120, wherein the display displays one or more ultrasonic images obtained by the one or more ultrasound transducers.

122. The system of clause 102, further comprising a computing device connected to the software-defined radio, such that the computing device controls the one or more outputs of the software-defined radio.

123. The system of clause 122, wherein the computing device comprises a display, wherein the display displays data received by the one or more inputs of the software-defined radio.

124. The system of clause 123, wherein the display displays one or more ultrasonic images obtained by the one or more ultrasound transducers.

125. A method of conditioning one or more ultrasound signals comprising: receiving a first transmission signal from a software-defined radio; amplifying the first transmission signal, thereby forming an amplified transmission signal; forwarding, with a first multiplexer, the amplified transmission signal to one or more ultrasound transducers; receiving, with the first multiplexer, one or more received ultrasound signals from the one or more ultrasound transducers; and amplifying the one or more received ultrasound signals, thereby forming one or more amplified received ultrasound signals.

126. The method of clause 125, wherein amplification of the one or more received ultrasound signals is based on a gain ramp transmitted by the software-defined radio.

127. The method of clause 126, wherein the amplification is time dependent.

128. The method of clause 127, wherein amplification of the one or more received ultrasound signals is carried out by one or more low noise, single-ended, linear, general purpose variable gain amplifiers.

129. The method of clause 125, wherein amplification of the one or more received ultrasound signals is carried out by one or more low noise, single-ended, linear, general purpose variable gain amplifiers.

130. The method of clause 125, further comprising emitting one or more ultrasound waves toward at least one object, wherein the at least one object reflects the one or more ultrasound waves, and wherein the reflected ultrasound waves are detected by the one or more ultrasound transducers and form the one or more received ultrasound signals.

131. The method of clause 130, wherein amplification of the one or more received ultrasound signals is based on a gain ramp transmitted by the software-defined radio.

132. The method of clause 131, wherein the amplification is time dependent.

133. The method of clause 132, wherein amplification of the one or more received ultrasound signals is carried out by one or more low noise, single-ended, linear, general purpose variable gain amplifiers.

134. The method of clause 130, wherein the at least one object is a blood vessel of a subject.

135. The method of clause 134, wherein the blood vessel is a carotid artery.

**Claims**

1. A system for transmitting and receiving ultrasound signals comprising: a software-defined radio comprising one or more outputs and one or more inputs;

    an ultrasound signal processing circuit electrically coupled to the software-defined radio; and one or more ultrasound transducers electrically coupled to the ultrasound signal processing circuit,
    wherein the ultrasound signal processing circuit processes one or more ultrasound transmission signals from the one or more outputs of the software-defined radio and transmits processed ultrasound transmission signals to the one or more ultrasound transducers to produce ultrasound waves, and wherein the signal processing unit processes one or more received ultrasound signals from the one or more ultrasound transducers and transmits processed received ultrasound signals to the one or more inputs of the software-defined radio.

2. The system of claim 1, wherein the ultrasound processing circuit comprises at least one high voltage amplifier to amplify the one or more ultrasound transmission signals from the one or more outputs of the software-defined radio.

3. The system of claim 2, wherein the ultrasound processing circuit comprises at least one variable gain amplifier to amplify the one or more received ultrasound signals from the one or more ultrasound transducers.

4. The system of claim 1, wherein the outputs of the software-defined radio comprise a gain ramp and a transmission pulse, wherein the ultrasound processing circuit comprises a high voltage amplifier to amplify the transmission pulse.

5. The system of claim 4, wherein the ultrasound processing circuit comprises a variable gain amplifier to amplify the one or more received ultrasound signals from the one or more ultrasound transducers based on the gain ramp transmitted by the software-defined radio.

6. The system of claim 5, wherein a rate of amplification of the ramp signal corresponds to the time since an initial pulse of the ramp signal was emitted by the software-defined radio.

7. The system of claim 6, the ultrasound transducers comprise a first pixel group and a second pixel group, wherein the first pixel group and the second pixel group each comprise 16 pixels.

8. The system of claim 1, the ultrasound transducers comprise a first pixel group and a second pixel group.

9. The system of claim 8, wherein the first pixel group is configured to receive the processed ultrasound transmission signals.

10. The system of claim 1, further comprising a computing device connected to the software-defined radio, such that the computing device controls the one or more outputs of the software-defined radio.

11. The system of claim 119, wherein the computing device comprises a display, wherein the display displays data received by the one or more inputs of the software-defined radio, wherein the display displays one or more ultrasonic images obtained by the one or more ultrasound transducers.

12. A method of conditioning one or more ultrasound signals comprising:

    receiving a first transmission signal from a software-defined radio;
    amplifying the first transmission signal, thereby forming an amplified transmission signal;
    forwarding, with a first multiplexer, the amplified transmission signal to one or more ultrasound transducers;
    receiving, with the first multiplexer, one or more received ultrasound signals from the one or more ultrasound transducers; and
amplifying the one or more received ultrasound signals, thereby forming one or more amplified received ultrasound signals.

13. The method of claim 12, wherein amplification of the one or more received ultrasound signals is based on a gain ramp transmitted by the software-defined radio, wherein the amplification is time dependent.

14. The method of claim 13, wherein amplification of the one or more received ultrasound signals is carried out by one or more low noise, single-ended, linear, general purpose variable gain amplifiers.

15. The method of claim 12, further comprising emitting one or more ultrasound waves toward at least one object, wherein the at least one object reflects the one or more ultrasound waves, and wherein the reflected ultrasound waves are detected by the one or more ultrasound transducers and form the one or more received ultrasound signals.

16. The method of claim 15, wherein amplification of the one or more received ultrasound signals is based on a gain ramp transmitted by the software-defined radio, wherein the amplification is time dependent, and wherein amplification of the one or more received ultrasound signals is carried out by one or more low noise, single-ended, linear, general purpose variable gain amplifiers.

17. The method of claim 15, wherein the at least one object is a blood vessel of a subject, and wherein the blood vessel is a carotid artery.

FIG. 1

# FIG. 2

# FIG. 3

CONTINUOUS BLOOD PRESSURE MEASUREMENT DEVICE 300

ELECTROACOUSTIC TRANSDUCER 310

AUDIO SIGNAL GENERATOR 305

PROCESSING DEVICE 330

· · ·

ULTRASOUND SENSOR(S) 320

WIRELESS TRANSMITTER 315

NON-ULTRASOUND SENSOR(S) 360

MACHINE READABLE MEDIUM(S) 340

POWER CIRCUITRY 302

BATTERY 301

DISPLAY SYSTEM 350

WIRELESS RECEIVER 355

PROCESSING DEVICE 352

DISPLAY 354

MACHINE READABLE MEDIUM 356

EP 4 555 939 A2

FIG. 4

450

Soft
Tissue

Blood

Arterial
Wall

Echogenic
Boundaries

FIG. 5

## FIG. 6

600

610 — Place Ultrasonic Transducer And Electroacoustic Transducer Into Proximity Of Blood Vessel Of Subject

620 — Actuate Electroacoustic Transducer To Emit A Plurality Of Soundwaves Having A Plurality Of Frequencies, The Soundwaves Producing A Vibrational Response In The Blood Vessel

630 — Determine, Based On The Vibrational Response Of The Blood Vessel To The Plurality Of Soundwaves, A Resonant Frequency Of The Blood Vessel

640 — Determine, Using Ultrasonic Transducer, Wall Thickness And Radius Of The Blood Vessel

650 — Calculate, Based On The Resonant Frequency, Wall Thickness, And Radius, The Blood Pressure Of The Subject

FIG. 7B

FIG. 7A

## FIG. 8A

## FIG. 8B

## FIG. 8C

## FIG. 8D

805

FIG. 9

EP 4 555 939 A2

FIG. 10

FIG. 11

Sensor m + 1

Sensor m

· · ·

Sensor n

Signal Processing

Image Processing

Single-Pixel Feature Extraction

Multi-Pixel Image Synthesis

Multi-Pixel Feature Extraction

Output to Operator

Output to Operator

Image

FIG. 12

1300

| PROCESSOR 1304 | DSP 1308 | ASIC 1310 |

BUS 1302

MEMORY 1306

**REFERENCES CITED IN THE DESCRIPTION**

**Patent documents cited in the description**

- US 62966927